# EUROPEAN PATENT SPECIFICATION

(11) **EP 2 069 338 B1**
(45) Date of publication and mention of the grant of the patent: **28.11.2012**
(21) Application number: 07786486.6
(22) Date of filing: 01.08.2007
(51) Int. Cl.: C07D 413/10, C07D 413/14, A01N 43/80

(54) **INSECTICIDAL ISOXAZOLINES**
INSEKTIZIDE ISOXAZOLINE
ISOXAZOLINES INSECTICIDES

(30) Priority: 15.08.2006 JP 2006221370
(43) Date of publication of application: 17.06.2009
(60) Divisional application: 10197327.9
(73) Proprietor: Bayer CropScience AG, 40789 Monheim (DE)
(72) Inventor: MIHARA, Jun, Tochigi 323-0022 (JP); MURATA, Tetsuya, Tochigi 323-0807 (JP); YAMAZAKI, Daiei, Tochigi 323-0829 (JP); YONETA, Yasushi, Saitama 347-0056 (JP); SHIBUYA, Katsuhiko, Tochigi 329-0433 (JP); SHIMOJO, Eiichi, Tochigi 323-0820 (JP); GÖRGENS, Ulrich, 40882 Ratingen (DE)
(86) International application number: PCT/EP2007/006798
(87) International publication number: WO 2008/019760

(56) References cited:
- WO-A-2005/085216
- WO-A-2007/075459

## Description

The present invention relates to novel isoxazolines, processes for the preparation thereof, a use thereof as insecticides and new intermediates thereof, as well as their use for controlling animal parasites.

WO 2005/085216 describes that isoxazoline substituted benzamides are useful as pest-controlling agents.

There have now been found novel isoxazolines of the following formula (I) 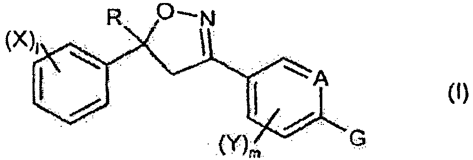 wherein:
- A: represents C or N;
- R: represents C₁₋₄ haloalkyl;
- X: represents the same or different halogen C₁₋₄ haloalkyl;
- 1: represents 0, 1 or 2;
- Y: represents ,
- m: represents 1 or 2; and
- G: represents any one selected from heterocyclic groups represented by the formulae G-1 to G-9: 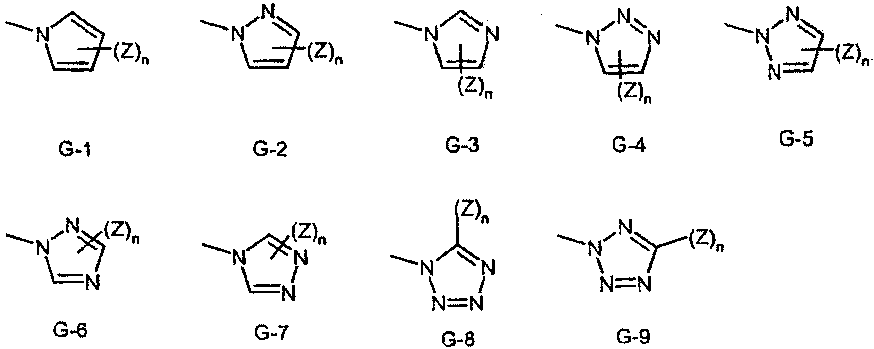
in which
- Z: represents halogen methyl, methylthio, trifluorormethyl cyano, nitro or amino; and
- n: represents 0 or 1.

The compounds of the formula (I), according to the present invention, may be obtained by a method in which
(a) compounds of the formula (II) 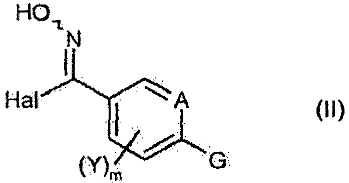 wherein A, Y, m and G have the same meaning as above and Hal represents halogen, are reacted with compounds of the formula (III) 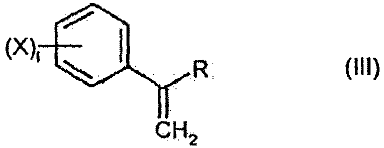 wherein R, X and 1 have the same meaning as above, in the presence of inert solvents, and if appropriate, in the presence of a base,
   or
(b) compounds of the formula (IV) 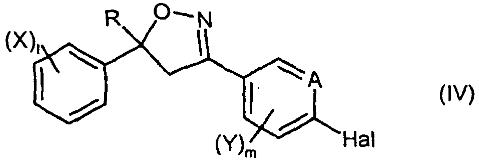 wherein A, R, X, 1, Y, m and Hal have the same meaning as above, are reacted with compounds of the formula (V)

   G-H (V)

   wherein G has the same meaning as above, in the presence of inert solvents, and if appropriate, in the presence of a base,
   or
(c) if G represents 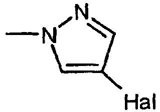 compounds of the formula (Ia) 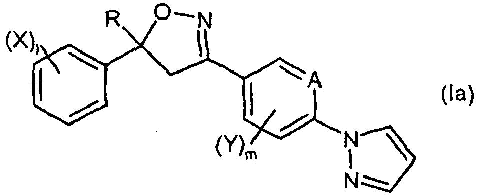 wherein A, R, X, 1, Y and m have the same meaning as above, are reacted with halogenating agents in the presence of inert solvents,
   or
(d) if G represents 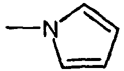 compounds of the formula (VI) 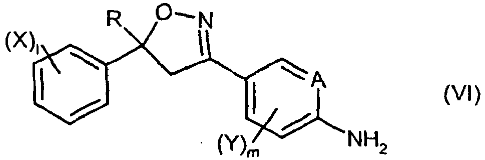 wherein A, R, X, 1, Y and m have the same meaning as above, are reacted with compounds of the formula (VII) 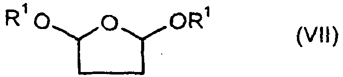 wherein R¹ represents alkyl, in the presence of inert solvents,
   or
(e) if G represents 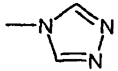 the compounds of the formula (VI) are reacted with 1,2-diformylhydrazine in the presence of a base,
   or
(f) if G represents 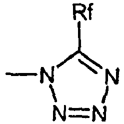 wherein Rf stands for perfluoroalkyl compounds of the formula (VIII) 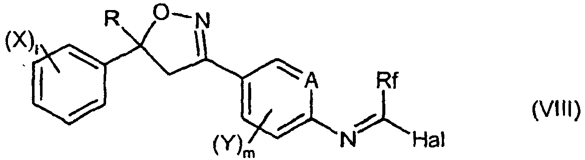 wherein A, R, X, 1, Y, m, Hal and Rf have the same meaning as above, are reacted with azide compounds in the presence of inert solvents,
   or
(g) if G represents 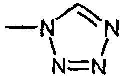 compounds of formula (VI) are reacted with azide compounds and trialkyl orthoformates in the presence of inert solvents,
   or
(h) in case where A represents C, and at least one of (Y)ₘ represents 3-NH₂ compounds of the formula (Ib) 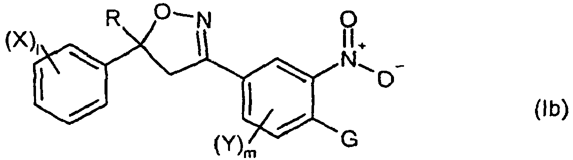 wherein R, X, 1, Y, m and G have the same meaning as above, are reduced in the presence of inert solvents,
   or
(i) in case where "A" represents C, and at least one of (Y)ₘ represents 3-NH-R², in which R² represents acyl, alkoxycarbonyl, haloalkoxycarbonyl or alkylsulfonyl:
compounds of the formula (Ic) 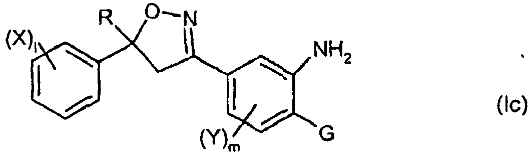 wherein R, X, 1, Y, m and G have the same meaning as above, are reacted with compounds of the formula (IX)

R²-T (IX)

wherein R² has the same meaning as above and T represents halogen or hydroxy,
in the presence of inert solvents, and, if appropriate, in the presence of a base.

According to the present invention, the isoxazolines of the formula (I) have a strong insecticidal activity. Moreover, it has been found that the novel compounds of formula (I) have pronounced biological properties and are suitable especially for controlling animal pests, in particular insects, arachnids and nematodes encountered in agriculture, in forests, in the protection of stored products and in the protection of materials, and also in the hygiene sector as well as in the veterinary field.

In this specification, the term "alkyl" means a straight-chain or branched C₁₋₁₂ alkyl such as methyl, ethyl, n- or iso-propyl, n-, iso-, sec- or tert-butyl, n-pentyl, n-hexyl, n-heptyl, n-octyl, n-nonyl, n-decyl, n-undecyl or n-dodecyl, preferably C₁₋₆ alkyl, and most preferably C₁₋₄ alkyl. An alkyl group is unsubstituted.

Examples of an alkyl moiety in each of the terms "alkoxy", "haloalkyl", "alkoxycarbonylamino", "haloalkoxycarbonylamino" and "alkylsulfonylamino" are those explained above for the "alkyl."

The term "acylamino" means, for example, alkylcarbonylamino, cyclopropylcarbonylamino and benzoyl, where examples of the alkyl moiety are those explained above for the "alkyl." An acylamino group can be unsubstituted, or substituted with at least one suitable substituent, selected from the substituents referred herein as Y.

The term "halogen" means fluorine, chlorine, bromine or iodine and preferably fluorine, chlorine or bromine.

Examples of the halogen moiety of the "haloalkyl" and "haloalkoxycarbonylamino" are those explained above for the "halogen."

Among the compounds of the formula (I) according to the present invention, preferable examples are those of the formula (I) in which
- A: represents C or N;
- R: represents C₁₋₄ haloalkyl;
- X: represents the same or different halogen or C₁₋₄ haloalkyl;
- 1: represents 0, 1 or 2;
- Y: represents
- m: represents 1 or 2; and
- G: represents any one selected from heterocyclic groups represented by the following formulae G-1 to G-9: 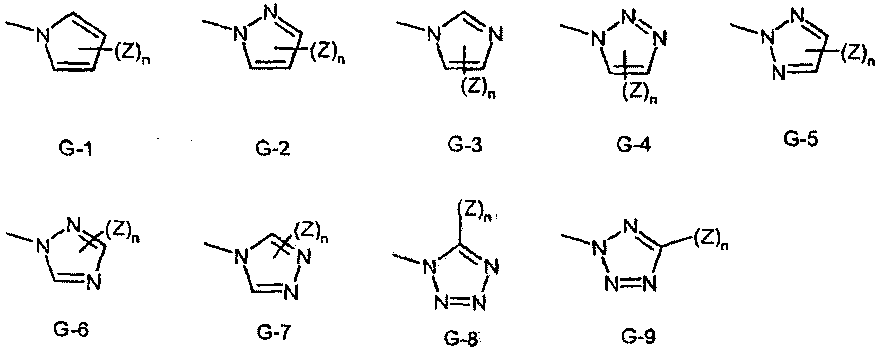 wherein

- Z: represents halogen, methyl, methylthio, trifluoromethyl, cyano, nitro or amino, and
- n: represents 0 or 1.

Among the compounds of the formula (I) according to the present invention, further preferable examples are those of the formula (I) in which
- A: represents C;
- R: represents optionally substituted C₁₋₁₂ haloalkyl;
- X: represents the same or different halogen or optionally substituted C₁₋₁₂ haloalkyl;
- 1: represents 0, 1 or 2;
- Y: represents cyano;
- m: represents 1 or 2; and
- G: represents any one selected from heterocyclic groups represented by the following formulae G-1 to G-9: 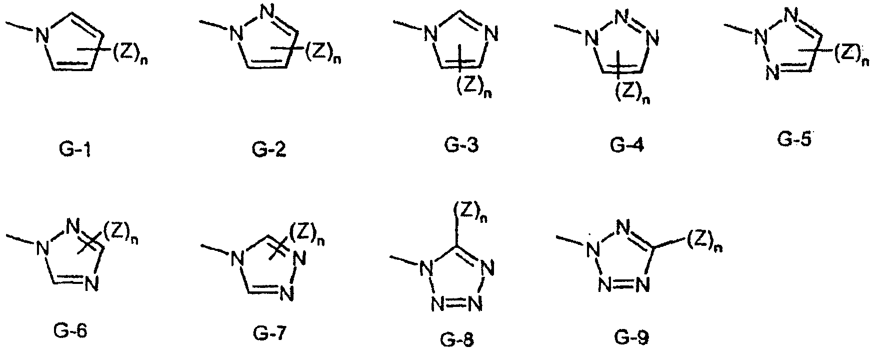
wherein
- Z: represents halogen, methyl, methylthio, trifluoromethyl, cyano, nitro or amino, and
- n: represents 0 or 1.

Moreover, among the compounds of the formula (I) according to the present invention, further preferable examples are those of the formula (I) in which
- A: represents N;
- R: represents optionally substituted C₁₋₁₂ haloalkyl;
- X: represents the same or different halogen or optionally substituted C₁₋₁₂ haloalkyl;
- l: represents 0, 1 or 2;
- Y: represents cyano;
- m: represents 1 or 2; and
- G: represents any one selected from heterocyclic groups represented by the following formulae G-1 to G-9: 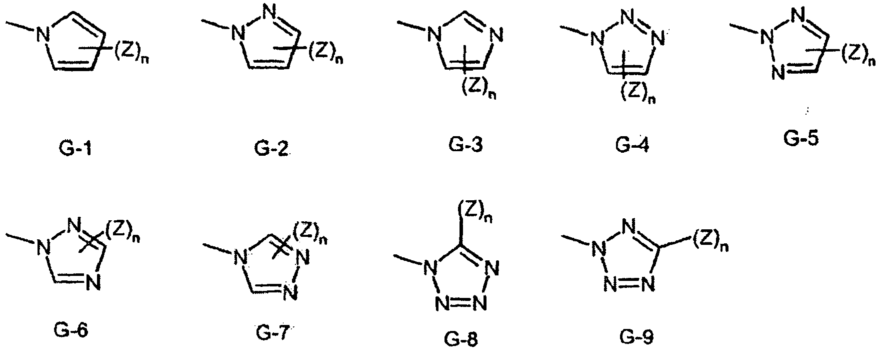
wherein
- Z: represents halogen, methyl, methylthio, trifluoromethyl, cyano, nitro or amino, and
- n: represents 0 or 1.

Among the compounds of the formula (I) according to the present invention, particularly preferable examples are those of the formula (I) in which;
- A: represents C or N;
- R: represents trifluoromethyl or pentafluoroethyl;
- X: represents the same or different fluoro, chloro, bromo or trifluoromethyl;
- t: represents 0, 1 or 2;
- Y: represents cyano;
- m: represents 1 or 2; and G represents any one of heterocyclic groups represented by the following formulae G-1 to G-9: 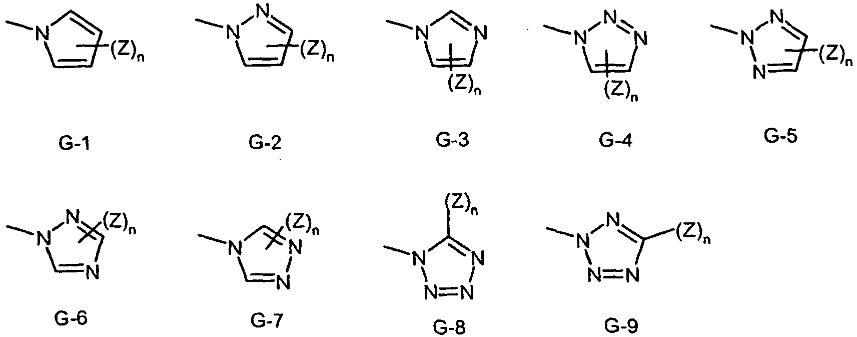
wherein
- Z: represents halogen, methyl, methylthio, trifluoromethyl, cyano, nitro or amino; and
- n: represents 0 or 1.

The compounds of the formula (I) according to the present invention have asymmetric carbons and therefore include optical or geometrical isomers or corresponding isomer mixtures of varying composition. The invention relates both to the pure isomers and the isomer mixtures.

The above preparation method (a) may be represented by the following reaction formula, when, for example, 3-cyano-N-hydroxy-4-(1H-1,2,4-triazol-1-yl)benzenecarboxyimidoyl chloride and 1,3-dichloro-5-[1-(trifluoromethyl)vinyl]benzene are used as starting materials.

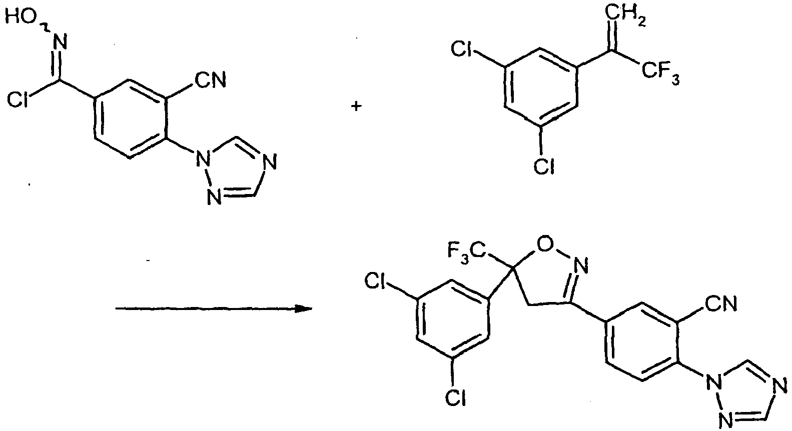

The above preparation method (b) may be represented by the following reaction formula, when, for example, 3-(4-fluoro-3-nitrophenyl)-5-(3,5-dichlorophenyl)-5-(trifluoromethyl)-4,5-dihydroisoxazole and I H-1,2,4-triazole are used as starting materials.

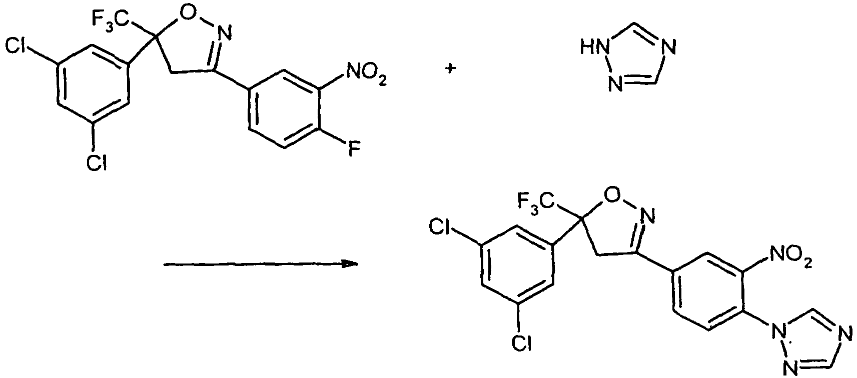

The above preparation method (c) may be represented by the following reaction formula, when, for example, 5-[5-(3,5-dichlorophenyl)-5-(trifluoro-methyl)-4,5-dihydroisoxazol-3-yl]-2-(1H-pyrazol-1-yl)-benzonitrile is used as a starting material and N-chlorosuceinimide is used as a halogenating agent.

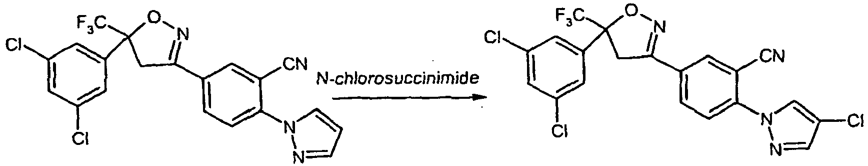

The above preparation method (d) may be represented by the following reaction formula, when, for example, 4-[5-(3,5-dichlorophenyl)-5-(trifluoro-methyl)-4,5-dihydroisoxazol-3-yl]aniline and 2,5-dimethoxy-tetrahydrofuran are used as starting materials.

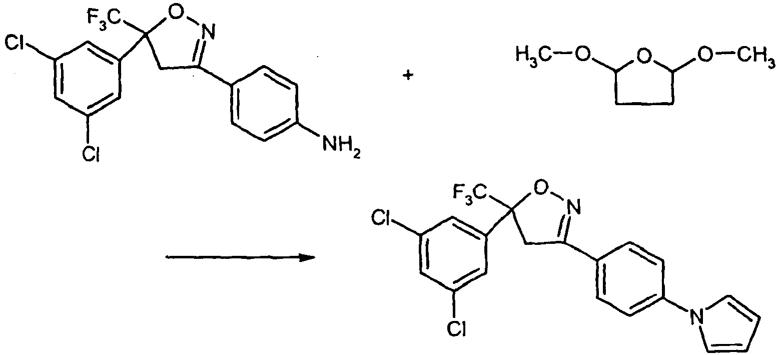

The above preparation method (e) may be represented by the following reaction formula, when, for example, 4-[5-(3,5-dichlorophenyl)-5-(trifluoro-methyl)4,5-dihydroisoxazol-3-yl] aniline and 1,2-diformyl-hydrazine are used as starting materials.

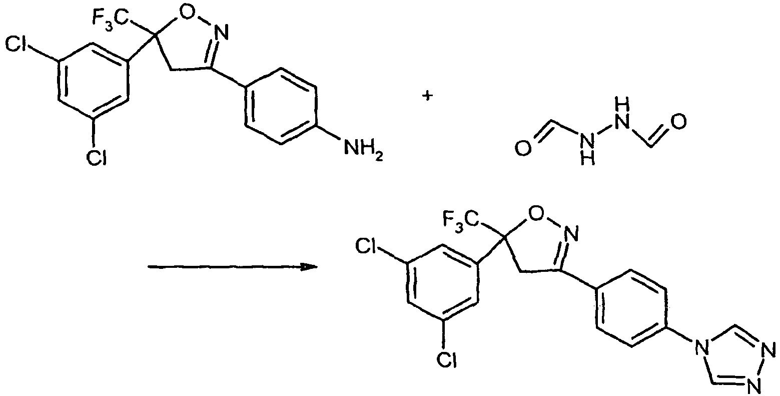

The above preparation method (f) may be represented by the following reaction formula, when, for example, N-{4-[5-(3,5-dichlorophenyl)-5-(trifluoromethyl)-4,5-dihydroisoxazo1-3-yl]phenyl}-2,2,2-trifluoroethanimidoyl chloride and sodium azide are used as starting materials.

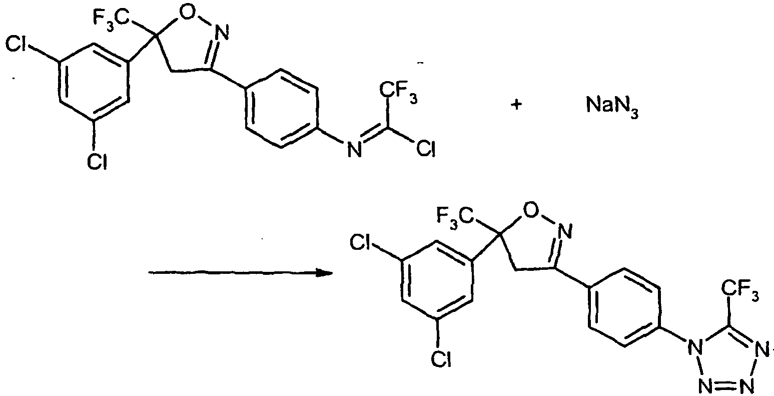

The above preparation method (g) may be represented by the following reaction formula, when, for example, 4-[5-(3,5-dichlorophenyl)-5-(trifluoro-methyl)-4,5-dihydroisoxazol-3-yl]aniline, ethyl orthoformate and sodium azide are used as starting materials.

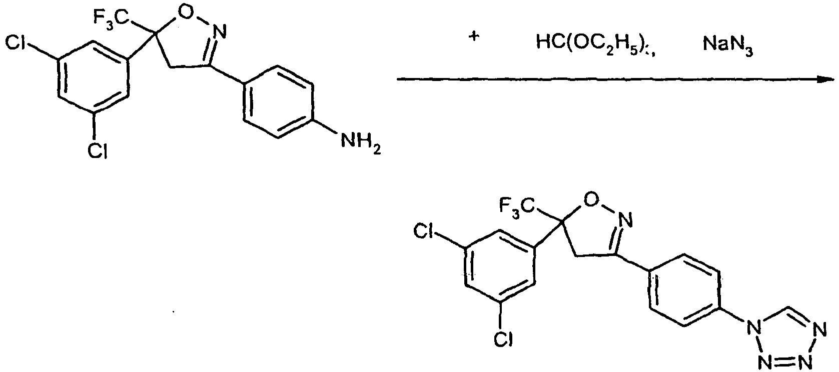

The above preparation method (h) may be represented by the following reaction formula, when, for example, 1-{4-[5-(3,5-dichlorophenyl)-5-(tri-fluoromethyl)-4,5-dihydroisoxazol-3-yl]-2-nitrophenyl}-1H-1,2,4-triazole is used as a starting material and reduced.

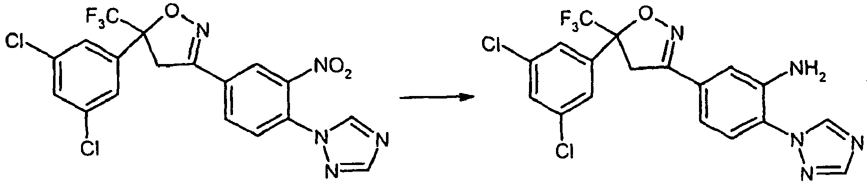

The above preparation method (i) may be represented by the following reaction formula, when, for example, 5-[5-(3,5-dichlorophenyl)-5-(trifluoro-methyl)-4,5-dihydroisoxazol-3-yl]-2-(1H-1,2,4-triazol-1-yl)aniline and acetyl chloride are used as starting materials.

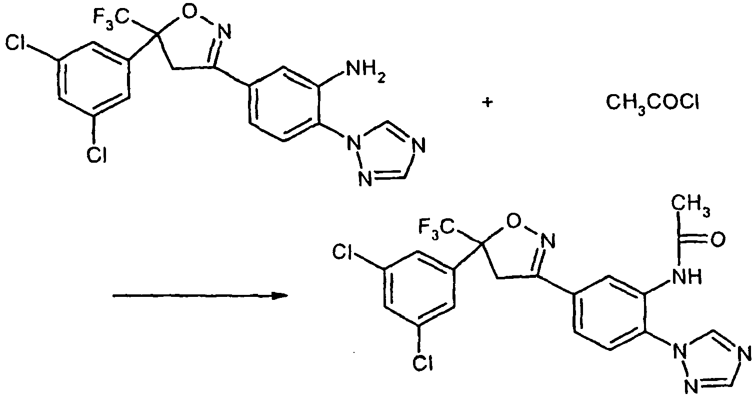

The starting material used in the preparation method (a), namely the compounds of the formula (II) starting are novel compounds, and can be obtained by reacting compounds of the formula (X) 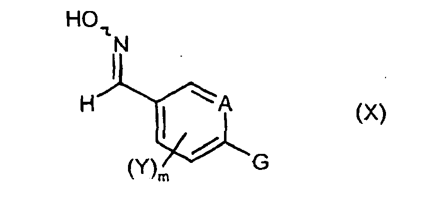
wherein A, Y, m and G have the same meaning as above with halogenating agents.

Compounds of the above formula (X) can be obtained by reacting compounds of the formula (XI) 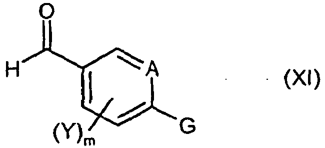 wherein A, Y, m and G have the same meaning as above, with hydroxylamine or salts thereof.

Compounds of the formula (XI) can be obtained by reacting, for example, compounds of the formula (XII) 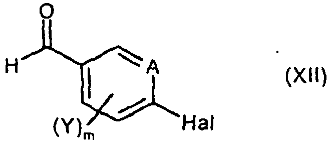 wherein A, Y, m and Hal have the same meaning as above, with compounds of formula (V).

The compounds of formula (XII) are well known and examples thereof include:
4-fluorobenzaldehyde, 3,4-difluorobenzaldehyde, 2-chloro4-fluorobenzaldehyde, 3-chloro-4-fluorobenzaldehyde, 3-bromo-4- fluorobenzaldehyde, 4-fluoro-3-iodobenzaldhyde, 4-fluoro-3-methylbenzaldhyde, 4-fluoro-3-trifluoromethylbenzaldehyde, 2-fluoro-5-formylbenzonitrile, and 3-chloronicotinaldehyde.

The aforementioned aldehydes may be synthesized, for example, according to the method described in Journal of Medicinal Chemistry, 2003, vol. 46, pp. 4232-4235.

Known examples of the compounds of formula (XI) are known and include:
4-(1H-pyrazol-1-yl)benzaldehyde, 4-(1H-imidazol-1-yl)benzaldehyde, 4-(1H-1,2,3-triazol-1-yl)benzaldehyde, and 4-(1H-1,2,5-triazol-1-yl)benzaldehyde. These compounds are described in Journal of Medicinal Chemistry, 1998, vol. 41, pp. 2390-2410. 6-(1H-imidazol-1-yl)-nicotinaldehyde (described in WO 88/00468A), 3-fluoro-4-(1H-imidazol-1-yl)benzaldehyde and 3-chloro-4-(1H-imidazol-1-yl)benzaldehyde (which are described in WO 2005/115990A); 3-bromo-4-(1H-pyrrol-1-yl)benzaldehyde and 3-bromo-4-(1H-imidazol-1-yl)benzaldehyde which are described in WO 2005/016862A; 3-fluoro-4-(1H-pyrazol-1-yl)benzaldehyde and 3-fluoro-4-(1H-1,2,4-triazol-1-yl)benzaldehyde which are described in WO 2002/046204A.

Preferable examples of novel compounds among the compounds of the formula (XI) include:
5-formyl-2-(1H-1,2,4-triazol-1-yl)benzonitrile,
5-formyl-2-(4-nito-1H-pyrazol-1-yl)benzonitrile and
5-formyl-2-(1H-tetrazol-1-yl)benzonitrile.

Many compounds of the formula (X) are novel and not described in the prior art. This is also true for the compounds of the following formula (Xa) 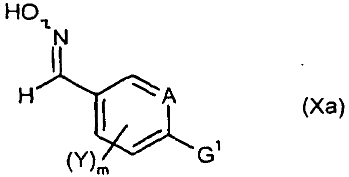 wherein A, Y and m have the same meaning as above and G¹ has the same meaning as G, provided that G¹ does not represent 1H-imidazol-1-yl when A represents C and m represents 0. N-[4-(1H-imidazol-1-yl)-phenyl]hydroxylamine has been described in WO 95/29163A.

Typical examples of the compounds of the formula (X) include: 3-bromo-4-(4-nitro-1H-pyrazol-1-yl)benzaldehyde oxime, 3-bromo-4-(4-cyano-1H-pyrazol-1-yl)benzaldehyde oxime, 5-[(hydroxyimino)methyl]-2-(4-nitro-1H-pyrazol-1-yl)benzonitrile, 5-[(hydroxyimino)methyl]-2-(4-cyano-1H-pyrazol-1-yl)benzonitrile, 4-(1H-1,2,4-triazol-1-yl)benzaldehyde oxime, 3-chloro-4-(IH-1,2,4-triazol-1-yl)benzaldehyde oxime, 3-bromo-4-(1H-1,2,4-triazol-1-yl)benzaldehyde oxime, 3-methyl-4-(1H-1,2,4-triazol-1-yl)benzaldehyde oxime, 4-(1H-1,2,4-triazol-1-yl)-3-trifluoromethyl-benzaldehyde oxime, 5-[(hydroxyimino)methyl]-2-(1H-1,2,4-triazol-1-yl)-benzonitrile, 6-(1H-1,2,4-triazol-1-yl)nicotinaldehyde oxime and 5-[(hydroxyimino)methyl]-2-(1H-tetrazol-1-yl)-benzonitrile.

The compounds of the formulae (II), (X) and (Xa) include optical or geometrical isomers or corresponding isomer mixtures of varying composition. The invention relates both to the pure isomers and the isomer mixtures.

Also, the halogenating agent usable in the preparation of the compounds of the formula (II) are generally known to the skilled person and include for example chlorine, bromine, iodine, N-chlorosuccinimide, N-bromosuccinimide, N-iodosuccinimide, 1,3-dichloro-5,5-dimethylhydantoin, 1,3-dibromo-5,5-dimethylhydantoin, benzyltrimethylammonium tetrachloroiodate and sodium hypochlorite.

Typical compounds of the formula (II), which is the starting material in the preparation method (a), include for example:
3-bromo-N-hydroxy-4-(4-nitro-1H-pyrazol-1-yl)-benzenecarboxyimidoyl chloride, 3-bromo-N-hydroxy-4-(4-cyano-1H-pyrazol-1-yl-benzenecarboxyimidoyl chloride, 3-cyano-N-hydroxy-4-(4-nitro-1H-pyrazol-1-yl)-benzenecarboxyimidoyl chloride, 3-cyano-N-hydroxy-4-(4-cyano-1H-pyrazol-1-yl)-benzenecarboxyimidoyl chloride, N-hydroxy-4-(1H-1,2,4-triazol-1-yl)-benzenecarboxy-imidoyl chloride, 3-chloro-N-hydroxy-4-(1H-1,2,4-triazol-1-yl-benzenecarboxyimidoyl chloride, 3-bromo-N-hydroxy-4-(1H-1,2,4-triazol-1-yl)-benzenecarboxyimidoyl chloride, N-hydroxy-3-methyl-4-(1H-1,2,4-triazol-1-yl)-benzenecarboxyimidoyl chloride, N-hydroxy-4-(1H-1,2,4-triazol-1-yl)-3-trifluoro-methylbenzenecarboxyimidoyl chloride, 3-cyano-N-hydroxy-4-(1H-1,2,4-triazol-1-yl)-benzenecarboxyimidoyl chloride, N-hydroxy-6-(1H-1,2,4-triazol-1-yl)pyridine-3-carboxyimidoyl chloride and 3-cyano-N-hydroxy-4-(1H-tetrazol-1-yl)-benzene-carboxyimidoyl chloride.

Compounds of formula (III) which are used as the other starting materials in the preparation method (a) include known compounds and which are for example described in Journal of Organic Chemistry, 1991, vol. 56, pp. 7336-7340; ditto 1994, vol. 59, pp. 2898-2901; and ditto, 1999, vol. 95, pp. 167-170; and WO 2005/05085216A.

Also, the compounds of the formula (III) may be synthesized by the methods described in these publications.

Typical examples of the compounds of the formula (III) include:
[1-(trifluoromethyl)vinyl]benzene, 1,3-difluoro-5-[1-(trifluoromethyl)vinyl]benzene, 1-chloro-3-[1-(trifluoromethyl)vinyl]benzene, 1,3-dichloro-5-[1-(trifluoromethyl)vinyl]benzene, 1-trifluoromethyl-3-[1-(trifluoromethyl)vinyl]-benzene, 1-trifluoromethyl-4-[1-(trifluoromethyl)vinyl]-benzene and 1,3-bis(trifluoromethyl)-5-[1-(trifluoromethyl)-vinyl]benzene.

The preparation method (a) may be carried out according to the methods described in, WO 2004/018410A, WO 2005/085216A, or Tetrahedron, 2000, vol. 56, pp. 1057-1064.

The reaction of the preparation method (a) may be carried out in an appropriate diluent or solvent. Examples thereof include aliphatic hydrocarbons (hexane, cyclohexane, heptane and others), aromatic hydrocarbons (benzene, toluene, xylene, chlorobenzene and others), alcohols (methanol, ethanol, isopropanol and others), ethers [diethyl ether, dibutyl ether, dimethoxyethane (DME), tetrahydrofuran, dioxane and others], acid amides [dimethylformamide (DMF), dimethylacetamide (DMA), N-methylpyrrolidone and others], nitriles (acetonitrile, propionitrile and others), dimethylsulfoxide (DMSO), water or mixtures of these solvents.

The reaction of the preparation method (a) may be carried out using a base, like for example an alkali metal base such as sodium carbonate, potassium carbonate, sodium bicarbonate, potassium bicarbonate, sodium acetate, potassium acetate, sodium methoxide, sodium ethoxide or potassium-tert-butoxide; an organic base such as triethylamine, diisopropylethylamine, tributylamine, N-methylmorpholine, N,N-dimethylaniline, N,N-diethylaniline, 4-tert-butyl-N,N-dimethylaniline, pyridine, picoline, lutidine, diazabicycloundecene, diazabicyclooctane or imidazole.

The reaction of the preparation method (a) may be carried out in a wide temperature range. The reaction may be carried out at a temperature in the range of usually about -78 to about 200°C and preferably -10 to about 150°C. Also, this reaction may be carried out under elevated pressure or under reduced pressure though it is preferably carried out under normal pressure. The reaction time is 0.1 to 72 hours, preferably 1 to 24 hours.

When the preparation method (a) is carried out, for example, a 1 to 2 molar amount of the compounds of formula (III) and 1 molar to a slightly excess amount of a base are reacted per mol of the compounds of the formula (II) in a diluent, like for example, DMF, yielding the aimed compounds of the formula (I).

The compounds of the formula (IV) which are the starting materials in the preparation method (b) are known and are, for example, described in, WO 2005/085216A.

Typical examples of the compounds of the formula (IV) include:
5-(3,5-dichlorophenyl)-3-(4-fluorophenyl)-5-(trifluoromethyl)-4,5-dihydroisoxazole, 3-(4-fluorophenyl)-5-[3-(trifluoromethyl)phenyl]-5-(trifluoromethyl)-4,5-dihydroisoxazole, 5-[3,5-bis(trifluoromethyl)phenyl]-3-(4-fluoro-phenyl)-5-(trifluoromethyl)-4,5-dihydroisoxazole, 3-(3-chloro-4-fluorophenyl)-5-(3,5-dichlorophenyl)-5-(trifluoromethyl)-4,5-dihydroisoxazole, 3-(3-chloro-4-fluorophenyl)-5-[3-(trifluoromethyl)-phenyl]-5-(trifluoromethyl)-4,5-dihydroisoxazole, 5-[3,5-bis(trifluoromethyl)phenyl]-3-(3-chloro-4-fluorophenyl)-5-(trifluoromethyl)-4,5-dihydroisoxazole, 3-(3-bromo-4-fluorophenyl)-5-(3,5-dichlorophenyl)-5-(trifluoromethyl)-4,5-dihydroisoxazole, 3-(3-bromo-4-fluorophenyl)-5-[3-(trifluoromethyl)-phenyl]-5-(trifluoromethyl)-4,5-dihydroisoxazole, 5-[3,5-bis(trifluoromethyl)phenyl]-3-(3-bromo-4-fluorophenyl)-5-(trifluoromethyl)-4,5-dihydroisoxazole, 5-[5-(3,5-dichlorophenyl)-5-(trifluoromethyl)-4,5-dihydroisoxazol-3-yl]-2-fluorobenzonitrile, 2-fluoro-5-{5-(trifluoromethyl)-5-[3-(trifluoromethyl)phenyl]-4,5-dihydroisoxazol-3-yl}-benzonitrile, 5-{5-[3,5-bis(trifluoromethyl)phenyl]-5-(trifluoro-methyl)-4,5-dihydroisoxazol-3-yl}-2-fluorobenzonitrile, 3-(4-fluoro-3-nitrophenyl)-5-(3,5-dichlorophenyl)-5-(trifluoromethyl)-4,5-dihydroisoxazole, 3-(4-chloro-3-nitrophenyl)-5-(3,5-dichlorophenyl)-5-(trifluoromethyl)-4,5-dihydroisoxazole, 3-(4-fluoro-3-nitrophenyl)-5-[3-(trifluoromethyl)-phenyl-5-(trifluoromethyl)-4,5-dihydroisoxazole, 5-[3,5-bis(trifluoromethyl)phenyl]-3-(4-fluoro-3-nitrophenyl)-5-(trifluoromethyl)-4,5-dihydroisoxazole, and 3-(6-chloropyridin-3-yl)-5-(3,5-dichlorophenyl)-5-(trifluoromethyl)-4,5-dihydroisoxazole.

The compounds of the formula (V) which are the starting materials of the preparation method (b) are well known in the fields of organic chemistry and typical examples thereof include:
1H-imidazole,1H-pyrazole, 4-methyl-1H-pyrazole, 4-fluoro-1H-pyrazole, 4-chloro- 1H-pyrazole, 4-bromo-1H-pyrazole,4-iodo-1H-pyrazole, 4-nitro-1H-pyrazole, 4-methyl-1H-pyrazole, 3-trifluoromethyl-1H-pyrazole, 4-trifluoromethyl-1H-pyrazole, 4-cyano-1H-pyrazole, 1H-1,2,3-triazole, 1H-1,2,4-triazole, 1H-tetrazole, 5-methyl-1H-tetrazole and 5-(methylthio)-1H-tetrazole.

Such azoles may be synthesized by the methods described in Journal of Medicinal Chemistry, 2005, vol. 48, pp. 5780-5793, Monatshefte für Chemie, 1993, vol. 124, pp. 199-207 and Tetrahedron Letters, 1996, vol. 37, pp. 1829-1832.

The reaction of the preparation method (b) may be carried out in an appropriate diluent or solvent. Examples thereof include:
aliphatic hydrocarbons (hexane, cyclohexane, heptane and others), aromatic hydrocarbons (benzene, toluene, xylene, chlorobenzene and others), ethers [diethyl ether, dibutyl ether and dimethoxyethane (DME), tetrahydrofuran, dioxane and others], acid amides [dimethylformamide (DMF), dimethylacetamide (DMA), N-methylpyrrolidone and others], nitriles (acetonitrile, propionitrile and others), dimethylsulfoxide (DMSO), water or mixtures of these solvents.

The reaction in the preparation method (b) may be carried out using a base, like for example an alkali metal base such as lithium hydride, sodium hydride, potassium hydride, lithium amide, sodium amide, lithium diisopropylamide, butyl lithium, tert-butyl lithium, trimethylsilyl lithium, lithium hexamethyldisilazide, sodium carbonate, potassium carbonate, sodium acetate, potassium acetate, sodium methoxide, sodium ethoxide or potassium-tert-butoxide or an organic base such as triethylamine, diisopropylethylamine, tributylamine, N-methylmorpholine, N,N-dimethylaniline, N,N-diethylaniline, 4-tert-butyl-N,N-dimethylaniline, pyridine, picoline, lutidine, diazabicycloundecene, diazabicyclooctane or imidazole.

The reaction of the preparation method (b) may be carried out in a wide temperature range. The reaction may be carried out at a temperature in the range of usually about -78 to about 200°C and preferably -10 to about 150°C. Also, this reaction may be carried out under elevated pressure or under reduced pressure though it is preferably carried out under normal pressure. The reaction time is 0.1 to 72 hours and preferably 1 to 24 hours.

When the preparation method (b) is carried out, for example, a 1 to 3 molar amount of the compounds of the formula (V) is reacted per mol of the compounds of the formula (IV) in the presence of I mol to 3 molar amount of a base in a diluent, like for example, DMF, yielding the aimed compounds of the formula (I).

The compounds of the formula (Ia) which are used as the starting materials in the preparation method (c) correspond to a part of the compounds of the formula (I).

Examples of the halogenating agent include the same compounds that are exemplified before.

The reaction of the preparation method (c) may be carried out in an appropriate diluent or solvent. Examples thereof include:
aliphatic hydrocarbons (hexane, cyclohexane, heptane and others), aromatic hydrocarbons (benzene, toluene, xylene, chlorobenzene and others), ethers [diethyl ether, dibutyl ether, dimethoxyethane (DME), tetrahydrofuran, dioxane and others], acid amides [dimethylformamide (DMF), dimethylacetamide (DMA), N-methylpyrrolidone and others], nitriles (acetonitrile, propionitrile and others), dimethylsulfoxide (DMSO) or mixtures of these solvents.

The reaction of the preparation method (c) may be carried out using a halogenating agent, like for example as chlorine, bromine, iodine, N-chlorosuccinimide, N-bromosuccinimide, N-iodosuccinimide, 1,3-dichloro-5,5-dimethylhydantoin, 1,3-dibromo-5,5-dimethylhydantoin or sodium hypochlorite.

The reaction of the preparation method (c) may be carried out in a wide temperature range. The reaction may be carried out at a temperature in the range of usually about -78 to about 200°C and preferably -10 to about 150°C. Also, this reaction may be carried out under elevated pressure or under reduced pressure though it is preferably carried out under normal pressure. The reaction time is 0.1 to 72 hours and preferably 0.1 to 24 hours.

When the preparation method (c) is carried out, for example, a 1 mol to a slightly excess amount of N-chlorosuccinimide is reacted per mol of the compounds of the formula (Ia) in a diluent, like for example, DMF, yielding the aimed compounds of the formula (I).

The compounds of the formula (VI) which are the starting materials of the preparation method (d) are known and described in, for example, WO 2005/085216A, and typical examples thereof include:
4-[5-(3,5-dichlorophenyl)-5-(trifluoromethyl)-4,5-dihydroisoxazol-3-yl] aniline, 2-chloro-4-[5-(3,5-dichlorophenyl)-5-(trifluoro-methyl)-4,5-dihydroisoxazol-3-yl]aniline, 2-bromo-4-[5-(3,5-dichlorophenyl)-5-(trifluoro-methyl)-4,5-dihydroisoxazol-3-yl]aniline, 4-{5-[3-(trifluoromethyl)phenyl]-5-(trifluoro-methyl)-4,5-dihydroisoxazol-3-yl) aniline, 2-chloro-4-{5-[3-(trifluoromethyl)phenyl]-5-(trifluoromethyl)-4,5-dihydroisoxazol-3-yl}aniline, 2-bromo-4-{5-[3-(trifluoromethyl)phenyl]-5-(trifluoromethyl)-4,5-dihydroisoxazol-3-yl}aniline, 4-{5-[3,5-bis(trifluoromethyl)phenyl]-5-(trifluoro-methyl)-4,5-dihydroisoxazol-3-yl}aniline, 2-chloro-4-{5-[3,5-bis(trifluoromethyl)phenyl]-5-(trifluoromethyl)-4,5-dihydroisoxazol-3-yl}aniline and 2-bromo-4-{5-[3,5-bis(trifluoromethyl)phenyl]-5-(trifluoromethyl)-4,5-dihydroisoxazol-3-yl}aniline.

The compounds of formula (VII) which are the starting materials of the preparation method (d) are well known compounds, and typical examples thereof include:
2,5-dimethoxytetrahydrofuran and 2,5-diethoxytetrahydrofuran.

The reaction in the preparation method (d) may be carried out in an appropriate diluent or solvent, and examples thereof include:
aliphatic hydrocarbons (hexane, cyclohexane and others), aromatic hydrocarbons (benzene, toluene, xylene, chlorobenzene and others), ethers [diethyl ether, dibutyl ether, dimethoxyethane (DME), tetrahydrofuran, dioxane and others], acid amides [dimethylformamide (DMF), dimethylacetamide (DMA), N-methylpyrrolidone and others], acids (acetic acid, and others), nitriles (acetonitrile, propionitrile and others), dimethylsulfoxide (DMSO) or mixtures of these solvents.

The preparation method (d) may be carried out in a wide temperature range. The reaction may be carried out at a temperature in the range of usually about 0 to about 200°C and preferably room temperature to about 150°C. Also, this reaction may be carried out under elevated pressure or under reduced pressure though it is preferably carried out under normal pressure. The reaction time is 0.1 to 72 hours and preferably 1 to 24 hours.

When the preparation method (d) is carried out, for example, a 1 mol to 5 molar amount of 2,5-dialkoxytetrahydrofuran is reacted per mol of the compounds of the formula (VI) in a diluent, like for example, acetic acid, yielding the aimed compounds of the formula (I).

The compounds of the formula (VI) which are the starting materials of the preparation method (e) are the same as those described in the above preparation method (d).

Also, the 1,2-diformylhydrazine which is the starting material is a known compound.

When the preparation method (e) is carried out, the compounds of the formula (VI) are reacted with 1,2-diformylhydrazine in the presence of a base and trialkylhalosilanes, whereby the corresponding compounds of the formula (I) can be obtained.

Specific examples of the trialkylhalosilanes may include: trimethylchlorosilane, triethylchlorosilane and trimethylbromosilane.

The preparation method (e) may be carried out according to the method described in The Journal of Organic Chemistry, 2001, vol. 44, pp. 3157-3165.

The reaction of the preparation method (e) may be carried out using a base, like for example an organic base such as triethylamine, diisopropylethylamine, tributylamine, N-methylmorpholine, N,N-dimethylaniline, N,N-diethylaniline, 4-tert-butyl-N,N-dimethylaniline, pyridine, picoline, lutidine, diazabicycloundecene, diazabicyclooctane or imidazole.

The reaction of the preparation method (e) may be carried out in a wide temperature range. The reaction may be carried out at a temperature in the range of usually about 0 to about 200°C and preferably about 0 to about 150°C. Also, this reaction may be carried out under elevated pressure or under reduced pressure though it is preferably carried out under normal pressure. The reaction time is 0.1 to 72 hours, preferably 1 to 24 hours.

When the preparation method (e) is carried out, for example, a 1 to 5 molar amount of 1,2-diformylhydrazine, 1 to 10 molar amount of a base and 1 to 25 molar amount of trialkylhalosilanes are reacted per mol of the compounds of formula (VI) in a great excess amount of pyridine, yielding the aimed compound of the formula (I).

The compounds of the formula (VIII) which are the starting materials in the preparation method (f) are novel compounds and are obtained by reacting compounds of the formula (XIII) 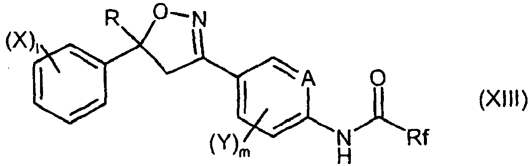 wherein A, R, X, 1, Y, m and Rf have the same meaning as above, with carbon tetrahalides and trivalent phosphorous compounds of the formula (XIV)

PL₃ (XIV)

wherein L represents C₄₋₈ alkyl or aryl.

The compounds of the formula (XIII) are also novel compounds and may be obtained by reacting the compounds of the formula (VI) with perfluoroalkylcarboxylic acid halides or perfluoroalkylcarboxylic acid anhydrides in the presence of a base.

The above carbon tetrahalides are known compounds. Specific examples of the carbon tetrahalides include carbon tetrachloride and carbon tetrabromide.The phosphorous compounds of the formula (XIV) are known, and specific examples thereof may include: tributylphosphine and triphenylphosphine.

The aforementioned perfluoroalkylcarboxylic acid halides or perfluoroalkylcarboxylic acid anhydrides are well-known compounds.

Examples of these compounds include trifluoroacetic acid anhydride, pentafluoropropionic acid anhydride and heptafluorobutyric acid anhydride.

Specific examples of the azide compounds to be used in the preparation method (f) include lithium azide and sodium azide.

The preparation method (f) may be carried out according to the method described in Japanese Patent Application (KOKAI) Publication No. 2005-154420A.

The reaction in the preparation method (f) may be carried out in an appropriate diluent or solvent, and examples thereof include:
aliphatic hydrocarbons (hexane, cyclohexane and others), aromatic hydrocarbons (benzene, toluene, xylene, chlorobenzene and others), ethers [diethyl ether, dibutyl ether, dimethoxyethane (DME), tetrahydrofuran, dioxane and others], amines (pyridine, collidine), acid amides [dimethylformamide (DMF), dimethylacetamide (DMA), N-methylpyrrolidone and others], nitriles (acetonitrile, propionitrile and others), dimethylsulfoxide (DMSO) or mixtures of these solvents.

The preparation method (f) may be carried out in a wide temperature range. The reaction may be carried out at a temperature in the range of usually about -78 to about 200°C and preferably room temperature i.e. 20°C to about 150°C. Also, this reaction may be carried out under elevated pressure or under reduced pressure though it is preferably carried out under normal pressure. The reaction time is 0.1 to 72 hours, preferably 1 to 24 hours.

When the preparation method (f) is carried out, for example, a 1 mol to 2 molar amount of an azide compound is reacted per mol of the compounds of the formula (VIII) in a diluent, for example, acetonitrile, yielding the aimed compounds of the formula (I).

The compounds of the formula (VI) which are the starting materials in the preparation method (g) are the same as those exemplified as the starting materials of the preparation method (d). Also, the azide compounds are the same as those mentioned in the above preparation method (f).

Moreover, the trialkyl orthoformates are known compounds, and specific examples thereof may include: trimethyl orthoformate and triethyl orthoformate.

The reaction in the preparation method (g) may be carried out in an appropriate diluent or solvent, and examples thereof include:
aliphatic hydrocarbons (hexane, cyclohexane and others), aromatic hydrocarbons (benzene, toluene, xylene, chlorobenzene and others), ethers [diethyl ether, dibutyl ether, dimethoxyethane (DME), tetrahydrofuran, dioxane and others], acid amides [dimethylformamide (DMF), dimethylacetamide (DMA), N-methylpyrrolidone and others], acids (acetic acid, propionic acid and others), nitriles (acetonitrile, propionitrile and others), dimethylsulfoxide (DMSO) or mixtures of these solvents.

The preparation method (g) may be carried out in a wide temperature range. The reaction may be carried out at a temperature in a range of usually about 0 to about 200°C and preferably room temperature, i.e. 20°C to about 150°C. Also, this reaction may be carried out under elevated pressure or under reduced pressure though it is preferably run under normal pressure. The reaction time is 0.1 to 72 hours, preferably 1 to 24 hours.

When the preparation method (g) is carried out, for example, a 1 to 3 molar amount of an azide compound and 1 to 10 molar amount of trialkyl orthoformate are reacted per mol of the compounds of the formula (VI) in a diluent, for example, acetic acid, yielding the aimed compounds of the formula (I).

The preparation method (g) may be carried out according to the method described in Journal of Medicinal Chemistry, 2000, vol. 43, pp. 953-970.

The compounds of the formula (Ib), as a part of the compounds of the formula (I), are included in the present invention.

Examples of the reduction reaction in the preparation method (h) include reactions using zinc, iron or stannous chloride; and hydrogenation reactions using catalysts, like palladium catalyst, nickel catalyst, cobalt catalyst, rhodium catalyst, ruthenium catalyst or platinum catalyst.

The reaction of the preparation method (h) may be carried out in an appropriate diluent or solvent, and examples thereof include:
aliphatic hydrocarbons (hexane, cyclohexane and others), aromatic hydrocarbons (benzene, toluene, xylene, chlorobenzene and others), ethers [diethyl ether, dibutyl ether and dimethoxyethane (DME), tetrahydrofuran, dioxane and others], acids (acetic acid, propionic acid and others), esters (ethyl acetate, ethyl propionate and others), water or mixtures of these solvents.

The preparation method (h) may be carried out in a wide temperature range. The reaction may be carried out at a temperature in the rang of usually about 0 to about 200°C and preferably room temperature, i.e. 20°C to about 150°C. Also, this reaction may be carried out under elevated pressure or under reduced pressure though it is preferably run under normal pressure. The reaction time is 0.1 to 72 hours, preferably 0.5 to 24 hours.

When the preparation method (h) is carried out, for example, a 3 to 5 molar amount of stannous chloride and a catalytic amount of concentrated hydrochloric acid are added per mol of the compounds of the formula (Ib) in a diluent, for example, ethanol, yielding the aimed compound of the formula (I).

The compounds of the formula (Ic), as a part of the compounds of the formula (I) are included into the present invention

Also, the compounds of the formula (IX) are known compounds, and typical examples thereof include:
acetyl chloride, propionyl chloride, isobutyryl chloride, cyclopropanecarbonyl chloride, benzoyl chloride, methyl chlorocarbonate, ethyl chlorocarbonate, methanesulfonyl chloride and benzoic acid.

The reaction of the preparation method (i) may be carried out in an appropriate diluent or solvent, and examples thereof include:
aliphatic hydrocarbons (hexane, cyclohexane and others), aromatic hydrocarbons (benzene, toluene, xylene, chlorobenzene and others), ethers [diethyl ether, dibutyl ether and dimethoxyethane (DME), tetrahydrofuran, dioxane and others], acid amides [dimethylformamide (DMF), dimethylacetamide (DMA), N-methylpyrrolidone and others], nitriles (acetonitrile, propionitrile and others), dimethylsulfoxide (DMSO), water or mixtures of these solvents.

The reaction in the preparation method (i) may be carried out using a base, like an alkali metal base such as for example lithium hydride, sodium hydride, potassium hydride, lithium amide, sodium amide, lithium diisopropylamide, butyl lithium, tert-butyl lithium, trimethylsilyl lithium, lithium hexamethyldisilazide, sodium carbonate, potassium carbonate, sodium acetate, potassium acetate, sodium methoxide, sodium ethoxide or potassium-tert-butoxide or an organic base such as triethylamine, diisopropylethylamine, tributylamine, N-methylmorpholine, N,N-dimethylaniline, N,N-diethylaniline, 4-tert-butyl-N,N-dimethylaniline, pyridine, picoline, lutidine, diazabicycloundecene, diazabicyclooctane or imidazole.

The reaction of the preparation method (i) may be carried out using, as a condensing agent, for example, 1,3-dicyclohexylcarbodiimide or 1-ethyl-3-(3'-dimethylamino-propyl)carbodiimide or a salt thereof.

When the preparation method (i) is carried out, for example, a 1 to 2 molar amount of the compounds of the formula (IX) is reacted per mol of the compounds of the formula (Ic) in the presence of 1 to 2 molar amount of a base in a diluent, for example, THF, yielding the aimed compounds of the formula (I).

The compounds of the formulae (II), (Xa), (VIII) and (XIII) which are used for the preparation of compounds of the formula (I), are novel compounds.

The formulae (VIII) and (XIII) can collectively be represented by the following formula (XV): 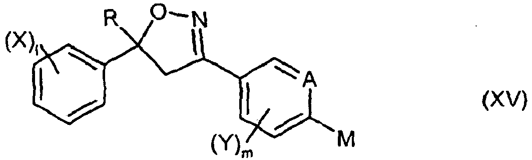 wherein A, R, X, 1, Y and m have the same meaning as defined herein, and M represents a group represented by the following formula: 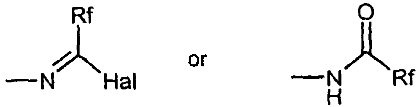 wherein Rf and Hal have the same meaning as defined herein.

The compounds of the formula (I), according to the present invention have strong insecticidal activity. Moreover the compounds according to the invention show strong activity against animal pests and thus may be used for controlling animal pests.

Accordingly, the compounds represented by the formula (I) according to the present invention *can* be used as insecticides, or for the manufacturing of a composition for controlling animal pests.

In the present invention, all compounds, compositions and materials having an insecticidal action on pests are called herein insecticides.

In addition, the active compounds represented by the formula (I) according to the present invention show an effect of controlling harmful insect specifically without imparting any phytotoxicity to cultivation plants.

Accordingly, the compounds of the present invention may be used to control a wide range of various animal pests In particular, to control sucking insects, chewing insects and other plant parasitic pests, stored-product pests and hygiene pests. Moreover, the compounds of the present invention may be applied to combat like e.g. exterminate and destroy these pests.

Examples of such pests include:
From Insecta, pests of Coleoptera, e.g., Callosobruchus Chinensis, Sitophilus zeamais, Tribolium castaneum, Epilachna vigintioctomaculata, Agriotes fuscicollis, Anomala rufocuprea, Leptinotarsa decemlineata, Diabrotica spp., Monochamus altematus, Lissorhoptrus oryzophilus, Lyctus bruneus and Aulacophora femoralis;
pests of Lepidoptera, e.g., Lymantria dispar, Malacosoma neustria, Pieris rapae, Spodoptera litura, Mamestra brassicae, Chilo suppressalis, Pyrausta nubilalis, Ephestia cautella, Adoxophyes orana, Carpocapsa pomonella, Agrotisfucosa, Galleria mellonella, Plutella maculipennis, Heliothis virescens and Phyllocnistis citrella;
pests of Hemiptera, e.g., Nephotettix cincticeps, Nilaparvata lugens, Pseudococcus comstocki, Unaspis yanonensis, Myzus persicas, Aphis pomi, Aphis gossypii, Phopalosiphum pseudobrassicas, Stephanitis nashi, Nazara spp. , Trialeurodes vaporariorm and Pshylla spp.;
pests of Thysanoptera, e.g., Thrips palmi and Franklinella occidental;
pests of Orthoptera, e.g., Blatella germanica, Periplaneta americana, Gryllotalpa africana and Locusta migratoria migratoriaodes;
pests of Isoptera, e.g., Reticulitermes speratus and Coptotermes formosanus; and
pests of Diptera, e.g., Musca domestica, Aedes aegypti, Hylemia platura, Culex pipiens, Anopheles sinensis, Culex tritaeniorhychus and Liriomyza trifolii;
and from Acarina, e.g., Tetranychus cinnabarinus, Tetranychus urticae, Panonychus citri, Aculops pelekassi and Tarsonemus spp.

Further examples of pests include:
From Nematoda, e.g., Meloidogyne incognita, Bursaphelenchus lignicolus Mamiya et Kiyohara, Aphelenchoides besseyi, Heterodera glycines and Pratylenchus spp.

Additionally, it has been found that the novel compounds of the present invention are active against animal parasites and thus may be effectively used for combating various harmful animal parasites (endo- and ectoparasites), like for example, in the veterinary field, insects and helminths.

Examples of such animal parasites may include the following pests:
From insects, e.g., Gastrophilus spp. Stomoxys spp. Trichodectes spp. Rhodnius spp. Ctenocephalidescanis, Cimx lecturius, Ctenocephalides felis and Lucila cuprina.
From Acarina, e.g., Omithodoros spp., Ixodes spp. and Boophilus spp.

As already mentioned before, in the veterinary fields, i.e. in the field of veterinary medicine, the active compounds according to the present invention are active against animal parasites (ecto- and endoparasites) such as hard ticks, soft ticks, scab mites, harvest mites, flies (stinging and licking), parasitic fly larvae, lice, hair lice, bird mites, bird lice and fleas.

These parasites include:
From the order of the Anoplurida, for example Haematopinus spp., Linognathus spp., Pediculus spp., Phtirus spp., Solenopotes spp.; particular examples are: Linognathus setosus, Linognathus vituli, Linognathus ovillus, Linognathus oviformis, Linognathus pedalis, Linognathus stenopsis, Haematopinus asini macrocephalus, Haematopinus eurysternus, Haematopinus suis, Pediculus humanus capitis, Pediculus humanus corporis, Phylloera vastatrix, Phthirus pubis, Solenopotes capillatus;
from the order of the Mallophagida and the suborders Amblycerina and Ischnocerina, for example Trimenopon spp., Menopon spp., Trinoton spp., Bovicola spp., Werneckiella spp., Lepikentron spp., Damalina spp., Trichodectes spp., Felicola spp.; particular examples are: Bovicola bovis, Bovicola ovis, Bovicola limbata, Damalina bovis, Trichodectes canis, Felicola subrostratus, Bovicola caprae, Lepikentron ovis, Werneckiella equi;
from the order of the Diptera and the suborders Nematocerina and Brachycerina, for example Aedes spp., Anopheles spp., Culex spp., Simulium spp., Eusimulium spp., Phlebotomus spp., Lutzomyia spp., Culicoides spp., Chrysops spp., Odagmia spp., Wilhelmia spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp., Rhinoestrus spp., Tipula spp.; particular examples are: Aedes aegypti, Aedes albopictus, Aedes taeniorhynchus, Anopheles gambiae, Anopheles maculipennis, Calliphora erythrocephala, Chrysozona pluvialis, Culex quinquefasciatus, Culex pipiens, Culex tarsalis, Fannia canicularis, Sarcophaga carnaria, Stomoxys calcitrans, Tipula paludosa, Lucilia cuprina, Lucilia sericata, Simulium reptans, Phlebotomus papatasi, Phlebotomus longipalpis, Odagmia ornata, Wilhelmia equina, Boophthora erythrocephala, Tabanus bromius, Tabanus spodopterus, Tabanus atratus, Tabanus sudeticus, Hybomitra ciurea, Chrysops caecutiens, Chrysops relictus, Haematopota pluvialis, Haematopota italica, Musca autumnalis, Musca domestica, Haematobia irritans irritans, Haematobia irritans exigua, Haematobia stimulans, Hydrotaea irritans, Hydrotaea albipuncta, Chrysomya chloropyga, Chrysomya bezziana, Oestrus ovis, Hypoderma bovis, Hypoderma lineatum, Przhevalskiana silenus, Dermatobia hominis, Melophagus ovinus, Lipoptena capreoli, Lipoptena cervi, Hippobosca variegata, Hippobosca equina, Gasterophilus intestinalis, Gasterophilus haemorroidalis, Gasterophilus inermis, Gasterophilus nasalis, Gasterophilus nigricornis, Gasterophilus pecorum, Braula coeca;
from the order of the Siphonapterida, for example Pulex spp., Ctenocephalides spp., Tunga spp., Xenopsylla spp., Ceratophyllus spp.; particular examples are: Ctenocephalides canis, Ctenocephalides felis, Pulex irritans, Tunga penetrans, Xenopsylla cheopis;
from the order of the Heteropterida, for example Cimex spp., Triatoma spp., Rhodnius spp., Panstrongylus spp.;
from the order of the Blattarida, for example Blatta orientalis, Periplaneta americana, Blattela germanica, Supella spp. (e.g. Suppella longipalpa);
from the subclass of the Acari (Acarina) and the orders of the Meta- and Mesostigmata, for example Argas spp., Omithodorus spp., Otobius spp., Ixodes spp., Amblyomma spp., Rhipicephalus (Boophilus) spp Dermacentor spp., Haemophysalis spp., Hyalomma spp., Dermanyssus spp., Rhipicephalus spp. (the original genus of multi host ticks) Ornithonyssus spp., Pneumonyssus spp., Raillietia spp., Pneumonyssus spp., Stemostoma spp., Varroa spp., Acarapis spp.; particular examples are: Argas persicus, Argas reflexus, Ornithodorus moubata, Otobius megnini, Rhipicephalus (Boophilus) microplus, Rhipicephalus (Boophilus) decoloratus, Rhipicephalus (Boophilus) annulatus, Rhipicephalus (Boophilus) calceratus, Hyalomma anatolicum, Hyalomma aegypticum, Hyalomma marginatum, Hyalomma transiens, Rhipicephalus evertsi, Ixodes ricinus, Ixodes hexagonus, Ixodes canisuga, Ixodes pilosus, Ixodes rubicundus, Ixodes scapularis, Ixodes holocyclus, Haemaphysalis concinna, Haemaphysalis punctata, Haemaphysalis cinnabarina, Haemaphysalis otophila, Haemaphysalis leachi, Haemaphysalis longicorni, Dermacentor marginatus, Dermacentor reticulatus, Dermacentor pictus, Dermacentor albipictus, Dermacentor andersoni, Dermacentor variabilis, Hyalomma mauritanicum, Rhipicephalus sanguineus, Rhipicephalus bursa, Rhipicephalus appendiculatus, Rhipicephalus capensis, Rhipicephalus turanicus, Rhipicephalus zambeziensis, Amblyomma americanum, Amblyomma variegatum, Amblyomma maculatum, Amblyomma hebraeum, Amblyomma cajennense, Dermanyssus gallinae, Omithonyssus bursa, Ornithonyssus sylviarum, Varroa jacobsoni;
from the order of the Actinedida (Prostigmata) and Acaridida (Astigmata), for example Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.; particular examples are: Cheyletiella yasguri, Cheyletiella blakei, Demodex canis, Demodex bovis, Demodex ovis, Demodex caprae, Demodex equi, Demodex caballi, Demodex suis, Neotrombicula autumnalis, Neotrombicula desaleri, Neoschöngastia xerothermobia, Trombicula akamushi, Otodectes cynotis, Notoedres cati, Sarcoptis canis, Sarcoptes bovis, Sarcoptes ovis, Sarcoptes rupicaprae (=S. caprae), Sarcoptes equi, Sarcoptes suis, Psoroptes ovis, Psoroptes cuniculi, Psoroptes equi, Chorioptes bovis, Psoergates ovis, Pneumonyssoidic Mange, Pneumonyssoides caninum, Acarapis woodi.

The active compounds of the formula (I) according to the invention are also suitable for controlling arthropods which attack agricultural livestock such as, for example, cattle, sheep, goats, horses, pigs, donkeys, camels, buffaloes, rabbits, chickens, turkeys, ducks, geese, honeybees, other domestic animals such as, for example, dogs, cats, cage birds, aquarium fish and what are known as experimental animals such as, for example, hamsters, guinea pigs, rats and mice.

By controlling these arthropods, it is intended to reduce deaths and improve performance (in the case of meat, milk, wool, hides, eggs, honey and the like), so that more economical and simpler animal keeping is made possible by the use of the active compound combinations according to the invention.

In the veterinary field and in animal keeping, they are applied in the known manner by enteral administration in the form of, for example, tablets, capsules, drinks, drenches, granules, pastes, boluses, the feed-through method, suppositories; by parenteral administration, such as, for example, by injections (intramuscular, subcutaneous, intravenous, intraperitoneal and the like), implants, by nasal application, by dermal application in the form of, for example, bathing or dipping, spraying, pouring-on and spotting-on, washing, dusting, and with the aid of active-compound-comprising shaped articles such as collars, ear tags, tail tags, limb bands, halters, marking devices and the like. The active compounds may be formulated as shampoo or as suitable formulations usable in aerosols, unpressurized sprays, for example pump sprays and atomizer sprays.

When used for livestock, poultry, domestic animals and the like, the active compounds of the formula (I) can be applied as formulations (for example powders, wettable powders ["WP"], emulsions, emulsifiable concentrates ["EC"], flowables, homogeneous solutions and suspension concentrates ["SC"]) which comprise the active compounds in an amount of from 1 to 80% by weight, either directly or after dilution (e.g. 100- to 10 000-fold dilution), or else as a chemical bath.

Furthermore, it has been found that the active compounds according to the invention have a potent insecticidal activity against insects which destroy industrial materials.

The following insects may be mentioned by way of example and by preference, but not by limitation:
Beetles such as
   Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.
Heminoptera such as
   Sirex juvencus, Urocerus gigas, Urocerus gigas taignus, Urocerus augur.
Termites such as
   Kalotermes flavicollis, Cryptotermes brevis, Heterotermes indicola, Reticulitermes flavipes, Reticulitermes saritonensis, Reticulitermes lucifugus, Mastotermes darwiniensis, Zootermopsis nevadensis, Coptotermes formosanus.
Bristletails such as Lepisma saccharina.
   Industrial materials are understood as meaning, in the present context, non-live materials such as, preferably, polymers, adhesives, glues, paper and board, leather, wood, derived timber products and paints.

If appropriate, a ready-to-use composition for the protection of industrial materials and which contain at least one compound according to the present invention, may additionally comprise further active ingredients, such as at least an insecticide and/or at least one fungicide. Suitable insecticides and/or fungizides are preferably those mentioned herein.

The compounds according to the invention can also be employed for protecting growths on objects, in particular ships' hulls, sieves, nets, buildings, moorings and signal systems which come into contact with salt water or brackish water.

Furthermore, the compounds according to the invention, alone or in combinations with further active ingredients, may be employed as antifouling agents.

In the hygiene sector, the active compounds according to the inventions are also found to be suitable for controlling animal pests, especially in the protection of domestic premises, in the field of hygiene and of stored products. They are found to be particularly active against insects, arachnids and mites which are found in enclosed spaces such as for example, dwellings, factory halls, offices, drivers' cabins and the like. To control these pests they can be used in insecticidal products for domestic premises, either alone or in combination with other active ingredients and auxiliaries. They are active against sensitive and resistant species and against all developmental stages.

Such animal pests include:
From the order of the Scorpionidea, for example Buthus occitanus;
from the order of the Acarina, for example Argas persicus, Argas reflexus, Bryobia ssp., Dermanyssus gallinae, Glyciphagus domesticus, Ornithodorus moubata, Rhipicephalus sanguineus, Trombicula alfreddugesi, Neutrombicula autumnalis, Dermatophagoides pteronissimus, Dermatophagoides forinae;
from the order of the Araneae, for example Aviculariidae, Araneidae;
from the order of the Opiliones, for example Pseudoscorpiones chelifer, Pseudoscorpiones cheiridium, Opiliones phalangium;
from the order of the Isopoda, for example Oniscus asellus, Porcellio scaber;
from the order of the Diplopoda, for example Blaniulus guttulatus, Polydesmus spp.;
from the order of the Chilopoda, for example Geophilus spp.;
from the order of the Zygentoma, for example Ctenolepisma spp., Lepisma saccharina, Lepismodes inquilinus;
from the order of the Blattaria, for example Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa;
from the order of the Saltatoria, for example Acheta domesticus;
from the order of the Dermaptera, for example Forficula auricularia;
from the order of the Isoptera, for example Kalotermes spp., Reticulitermes spp;
from the order of the Psocoptera, for example Lepinatus spp., Liposcelis spp;
from the order of the Coleoptera, for example Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum;
from the order of the Diptera, for example Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa;
from the order of the Lepidoptera, for example Achroia grisella, Galleria mellonella, Plodia interpunctella, Tinea cloacella, Tinea pellionella, Tineola bisselliella;
from the order of the Hymenoptera, for example Camponotus herculeanus, Lasius fuliginosus, Lasius niger, Lasius umbratus, Monomorium pharaonis, Paravespula spp., Tetramorium caespitum;
from the order of the Anoplura, for example Pediculus humanus capitis, Pediculus humanus corporis, Pemphigus spp., Phylloera vastatrix, Phthirus pubis;
from the order of the Heteroptera, for example Cimex hemipterus, Cimex lectularius, Rhodinus prolixus, Triatoma infestans.

In the hygiene sector, the application of the invention may be carried out alone or in combination with other suitable active ingredients, like for example insecticides or fungizides, preferably those mentioned herein, and active ingredients selected from phosphoric esters, carbamates, pyrethroids, neo-nicotinoids, growth regulators.

In generell, the application of the invention may be carried out in a manner fit to the application form. Suitable application forms include aerosols, unpressurized sprays, for example pump sprays and atomizer sprays, automatic misting devices, foggers, foams, gels, vaporizer products with vaporizer platelets made of cellulose or polymer, liquid vaporizers, gel and membrane vaporizers, propeller-driven vaporizers, vaporization systems which do not consume energy (passive vaporization systems), moth papers, moth sachets and moth gels in the form of granules or dusts, in baits for scattering or bait stations.

In particular, the active compounds of the present invention can be formulated into usual preparation forms. For the various applications, in particular in the agricultural field and hygiene sector, examples of the preparation forms, especially when used as insecticide, include solutions, emulsions, wettable powders, dry flowables, suspensions, dusts, foams, pastes, tablets, granules, aerosols, active compound infiltrated-natural and synthetic products, microcapsules, seed coating agents, preparations with a combustor (for example, fumigating and smoking cartridges, cans and coils), ULV (cold mists) and warm mists).

Each of these preparations may be prepared by a known manner per se. For example, at least one active compound is mixed with developers, specifically, liquid diluents or carriers; liquid gas diluents or carriers; or solid diluents or carriers, and optionally, with surfactants (like for example anionic, kationic and non-ionic surfactants), specifically, emulsifiers and/or dispersants and/or foam formers, thereby the formulations are prepared.

When water is used as the developer, for example, an organic solvent may also be used as an auxiliary solvent.

Examples of the liquid diluents or carriers may include aromatic hydrocarbons (for example, xylene, toluene and alkylnaphthalene), chlorinated aromatic or aliphatic hydrocarbons (for example, chlorobenzenes, ethylene chlorides and methylene chlorides), aliphatic hydrocarbons [for example, cyclohexane, paraffins (for example, mineral oil fractions)], alcohols (for example, benzyl alcohol, isopropanol, ethanol, butanol, glycol and ethers and esters thereof), ketones (for example, acetone, methyl ethyl ketone, methyl isobutyl ketone and cyclohexanone), strong polar solvents (for example, dimethylformamide and dimethylsulfoxide), cyclic carbonates (for example, ethylene carbonate, propylene carbonate), pyrrolidones (for example, N-octylpyrrolidone, N-methylpyrrolidone), ethers (for example, diethylene glycol monomethylether and diethylene glycol monopropylether), lactones (for example, butyrolacton) and water.

Examples of the liquid gas diluents or carriers may include those which are gas in normal temperature and pressure such as aerosol propellants such as fron, propane, nitrogen gas, carbon dioxide, and halogenated hydrocarbons.

Examples of the solid diluents may include ground natural minerals (for example, kaolins, clays, talc, chalk, quartz, attapulgite, montmorillonite or diatomaceous earth) and ground synthetic minerals (for example, highly dispersed silicic acid, alumina and silicate).

Examples of the solid carriers for granules may include crushed and fractionated rocks (for example, calcite, marble, pumice, sepiolite and dolomite), synthetic granules of inorganic or organic powders, organic materials (for example, sawdust, coconut shells, maize cobs and tobacco stalks).

Examples of the emulsifiers and/or foam formers may include nonionic and anionic emulsifiers [for example, polyoxyethylene fatty acid esters, polyoxyethylene fatty acid alcohol ethers (for example, alkylaryl polyglycol ether), alkyl sulfonates, alkyl sulfates and aryl sulfonates] and albumin hydrolysates.

The dispersants includes lignin sulfite waste liquor and methylcellulose.

Binders may also be used in the preparations (powders, granules and emulsifiable concentrates). Examples of the binder may include carboxymethylcellulose, natural or synthetic polymers (for example, gum arabic, polyvinyl alcohol and polyvinyl acetate).

Colorant may also be used in the present invention. Examples of the colorant may include inorganic pigments (for example, iron oxide, titanium oxide and Prussian blue), organic colorants such as Alizarin colorants, azo colorants or metal phthalocyanine colorants, and further, trace nutrients such as salts of iron, manganese, boron, copper, cobalt, molybdenum or zinc.

The preparation may contain the above active component in an amount range generally from 0.1 to 95% by weight of the total preparation and preferably 0.5 to 90% by weight.

The active compounds of the formula (I) of the present invention may exist as a mixture with other active compounds, for example, insecticides, poison baits, bactericides, acaricides, nematicides, fungicides, growth regulators or herbicides in the forms of preparations commercially useful and in the working forms prepared from these preparations. Here, examples of the above insecticides may include organic phosphorous agents, carbonate agents, carboxylate type chemicals, chlorinated hydrocarbon type chemicals and insecticidal materials produced from microorganisms. The active compounds of the formula (I) of the present invention may exist as a mixture with synergists, and examples of the preparation forms and working forms thereof may include commercially useful forms. The synergists themselves do not need to be active and are compounds enhancing the action of the active compounds.

Such active ingredients or synergists are, for example, the following:
Compounds acting as fungicides:
   Nucleic acid synthesis inhibitors, like Benalaxyl, benalaxyl-M, bupirimate, chiralaxyl, clozylacon, dimethirimol, ethirimol, furalaxyl, hymexazol, metalaxyl, metalaxyl-M, ofurace, oxadixyl and oxolinic acid.

Inhibitors of mitosis and cell division, like benomyl, carbendazim, diethofencarb, fuberidazole, pencycuron, thiabendazole, thiophanate-methyl and zoxamis.

Inhibitor of respiratory complex I, like diflumetorim.

Inhibitors of respiratory complex II, like boscalid, carboxin, fenfuram, flutolanil, furametpyr, mepronil, oxycarboxin, penthiopyrad and thifluzamide.

Inhibitors of respiratory complex III, like azoxystrobin, cyazofamide, dimoxystrobin, enestrobin, famoxadone, fenamidone, fluoxastrobin, kresoximmethyl, metominostrobin, orysastrobin, pyraclostrobin and picoxystrobin.

Decouplers, like dinocap and fluazinam.

Inhibitors of ATP production, like fentin acetate, fentin chloride, fentin hydroxide and silthiofam.

Inhibitor of amino acid and protein biosynthesis, like andoprim, blasticidin-S, cyprodinil, kasugamycin, kasugamycin hydrochloride hydrate, mepanipyrim, and pyrimethanil.

Inhibitors of signal transduction, like fenpiclonil, fludioxonil, and quinoxyfen

Inhibitors of fat and membrane synthesis, like chlozolinate, iprodione, procymidone, vinclozolin, ampropylfos, potassium ampropylfos, edifenphos, iprobenfos (IBP), isoprothiolane, pyrazophos, tolelofos-methyl, biphenyl, iodocarb, propamocarb, and propamocarb hydrochloride.

Inhibitors of ergosterol biosynthesis, like fenhexamide, azaconazole, bitertanol, bromuconazole, cyproconazole, diclobutrazole, difenoconazole, diniconazole, diniconazole-M, epoxiconazole, etaconazole, fenbuconazole, fluquinconazole, flusilazole, flutriafol, furconazole, furconazole-cis, hexaconazole, imibenconazole, ipconazole, metconazole, myclobutanil, paclobutrazole, penconazole, propiconazole, prothioconazole, simeconazole, tebuconazole, tetraconazole, triadimefon, triadimenol, triticonazole, uniconazole, voriconazole, imazalil, imazalil sulphate, oxpoconazole, fenarimol, flurprimidol, nuarimol, pyrifenox, triforin, pefurazoate, prochloraz, triflumizole, viniconazole, aldimorph, dodemorph, dodemorph acetate, fenpropimorph, tridemorph, fenpropidin, spiroxamine, naftifin, pyributicarb, and terbinafin.

Inhibitors of cell wall synthesis, like benthiavalicarb, bialaphos, dimethomorph, flumorph, iprovalicarb, polyoxins, polyoxorim, and validamycin A.

Inhibitors of melanin biosynthesis, like capropamide, diclocymet, fenoxanil, phtalide, pyroquilon, and tricyclazole.

Resistence induction compounds, like acibenzolar-S-methyl, probenazole, and tiadinil.

Multisite compounds, like captafol, captan, chlorothalonil, copper salts: copper hydroxide, copper naphthenate, copper oxychloride, copper sulphate, copper oxide, oxine-copper and Bordeaux mixture, dichlofluanid, dithianon, dodin, dodin freie base, ferbam, fluorofolpet, guazatin, guazatin acetate, iminoctadin, iminoctadine albesilate, iminoctadine triacetate, mancopper, mancozeb, maneb, metiram, metiram zinc, propineb, sulphur and sulphur preparations containing calcium polysulphide, thiram, tolylfluanid, zineb, and ziram.

Compounds of unknown mechanism, like amibromdol, benthiazole, bethoxazin, capsimycin, carvone, quinoline methionate, chloropicrin, cufraneb, cyflufenamide, cymoxanil, dazomet, debacarb, diclomezine, dichlorophen, dicloran, difenzoquat, difenzoquat methyl sulphate, diphenylamine, ethaboxam, ferimzone, flumetover, flusulfamide, fluopicolide, fluoroimide, hexachlorobenzene, 8-hydroxyquinoline sulphate, immamycin, methasulphocarb, metrafenone, methyl isothiocyanate, mildiomycin, natamycin, nickel dimethyldithiocarbamate, nitrothalisopropyl, octhilinone, oxamocarb, oxyfenthiin, pentachlorophenol and salts, 2-phenylphenol and salts, piperalin, propanosin -sodium, proquinazid, pyrrolnitrin, quintozen, tecloftalam, tecnazen, triazoxido, trichlamide, zarilamide and 2,3,5,6-tetrachloro-4-(methylsulphonyl)pyridine, N-(4-chloro-2-nitrophenyl)-N-ethyl-4-methylbenzenesulphonamide, 2-amino-4-methyl-N-phenyl-5-thiazole carboxamide, 2-chloro-N-(2,3-dihydro-1,1,3-trimethyl-1H-inden-4-yl)-3-pyridine carboxamide, 3-[5-(4-chlorophenyl)-2,3-dimethylisoxazolidin-3-yl]pyridine, cis-1-(4-chlorophenyl)-2-(1H-1,2,4-triazol-1-yl)cycloheptanol, 2,4-dihydro-5-methoxy-2-methyl-4-[[[[1-[3-(trifluoromethyl)-phenyl]ethyliden]amino]oxy]methyl]phenyl]-3H-1,2,3-triazol-3-one (185336-79-2), methyl 1-(2,3-dihydro-2,2-dimethyl-1H-inden-1-yl)-1H-imidazole-5-carboxylate, 3,4,5-trichloro-2,6-pyridine dicarbonitriel, methyl 2-[[[cyclopropyl[(4-methoxyphenyl) imino]methyl]thio]methyl]-alpha-(methoxymethylen)-benzacetate, 4-chloro-alpha-propinyloxy-N-[2-[3-methoxy-4-(2-propinyloxy)phenyl]ethyl]-benzacetamide, (2S)-N-[2-[4-[[3-(4-chlorophenyl)-2-propinyl]oxy]-3-methoxyphenyl]ethyl]- 3-methyl-2-[(methylsulphonyl)amino]-butanamide, 5-chloro-7-(4-methylpiperidin-1-yl)-6-(2,4,6-trifluorophenyl)[1,2,4]triazolo[1,5-a]pyrimidine, 5-chloro-6-(2,4,6-trifluorophenyl)-N-[(1R)-1,2,2-trimethylpropyl][1,2,4]triazolo[1,5-a]pyrimidine-7-amine, 5-chloro-N-[(1R)-1,2-dimethylpropyl]-6-(2,4,6-trifluorophenyl) [1,2,4]triazolo[1,5-a]pyrimidine-7-amine, N-[1-(5-bromo-3-chloropyridin-2-yl)ethyl]-2,4-dichloronicotinamide, N-(5-bromo-3-chloropyridin-2-yl)methyl-2,4-dichloronicotinamide, 2-butoxy-6-iodo-3-propylbenzopyranon-4-one, N-{(Z)-[(cyclopropylmethoxy) imino][6-(difluoromethoxy)-2,3-difluorophenyl]methyl}-2-benzacetamide, N-(3-ethyl-3,5,5-trimethylcyclohexyl)-3-formylamino-2-hydroxybenzamide, 2-[[[[1-[3(1fluoro-2-phenylethyl)oxy]phenyl ethylidene]amino]oxy]methyl]-alpha-(methoxyimino)-N-methyl-alphaE-benzacetamide, N-{2-[3-chloro-5-(trifluoromethyl)pyridin-2-yl]ethyl}-2-(trifluoromethyl)benzamide, N-(3',4'-dichloro-5-fluorobiphenyl-2-yl)-3-(difluoromethyl)-1-methyl-1H-pyrazole-4-carboxamide, N-(6-Methoxy-3-pyridinyl)-cyclopropane carboxamide, 1-[(4-methoxyphenoxy)methyl]-2,2-dimethylpropyl-1H-imidazole-1- carboxylic acid, O-[1-[(4-methoxyphenoxy)methyl]-2,2-dimethylpropyl]-1H-imidazole- 1- carbothioic acid, 2-(2-{[6-(3-chlor-2-methylphenoxy)-5-fluoropyrimidin-4-yl]oxy}phenyl)-2-(methoxyimino)-N-methylacetamide

Compounds acting as Bactericides, such as bronopol, dichlorophen, nitrapyrin, nickel dimethyldithiocarbamate, kasugamycin, octhilinon, furan carboxylic acid, oxytetracyclin, probenazol, streptomycin, tecloftalam, copper sulphate and other copper preparations.

Compounds acting as Insecticides and/or Acaricides and/or Nematicides:
Acetylcholinesterase (AChE) inhibitors, like carbamates, such as for example alanycarb, aldicarb, aldoxycarb, allyxycarb, aminocarb, bendiocarb, benfuracarb, bufencarb, butacarb, butocarboxim, butoxycarboxim, carbaryl, carbofuran, carbosulfan, cloethocarb, dimetilan, ethiofencarb, fenobucarb, fenothiocarb, formetanate, furathiocarb, isoprocarb, metam-sodium, methiocarb, methomyl, metolcarb, oxamyl, pirimicarb, promecarb, propoxur, thiodicarb, thiofanox, trimethacarb, XMC, xylylcarb, and triazamate; and
like organophosphates, such as for example acephate, azamethiphos, azinphos (-methyl, -ethyl), aromophos-ethyl, aromfenvinfos (-methyl), autathiofos, cadusafos, carbophenothion, chlorethoxyfos, chlorfenvinphos, chlormephos, chlorpyrifos (-methyl/-ethyl), coumaphos, cyanofenphos, cyanophos, chlorfenvinphos, demeton-S-methyl, demeton-S-methylsulphone, dialifos, diazinone, dichlofenthione, dichlorvos/DDVP, dicrotophos, dimethoate, dimethylvinphos, dioxabenzofos, disulfoton, EPN, ethion, ethoprophos, etrimfos, famphur, fenamiphos, fenitrothion, fensulfothion, fenthion, flupyrazofos, fonofos, formothion, fosmethilan, fosthiazate, heptenophos, iodofenphos, iprobenfos, isazofos, isofenphos, isopropyl O-salicylate, isoxathion, malathion, mecarbam, methacrifos, methamidophos, methidathion, mevinphos, monocrotophos, naled, omethoate, oxydemeton-methyl, parathion (-methyl/-ethyl), phenthoate, phorate, phosalone, phosmet, phosphamidone, phosphocarb, Phoxim, pirimiphos (-methyl/-ethyl), profenofos, propaphos, propetamphos, prothiofos, prothoate, pyraclofos, pyridaphenthion, pyridathion, quinalphos, sebufos, sulfotep, sulprofos, tebupirimfos, temephos, terbufos, tetrachlorvinphos, thiometon, triazophos, triclorfon, vamidothion.
Sodium channel modulators / voltage-dependent sodium channel blockers like pyrethroids, such as for example acrinathrin, allethrin (d-cis-trans, d-trans), beta-cyfluthrin, bifenthrin, bioallethin, bioallethrin-S-cyclopentyl-isomer, bioethanomethrin, biopermethrin, bioresmethrin, chlovaporthrin, cis-cypermethrin, cis-resmethrin, cis-permethrin, clocythrin, cycloprothrin, cyfluthrin, cyhalothrin, cypermethrin (alpha-, beta-, theta-, zeta), cyphenothrin, deltamethrin, empenthrin (1R-isomer), esfenvalerate, etofenprox, fenfluthrin, fenpropathrin, fenpyrithrin, fenvalerate, flubrocythrinate, flucythrinate, flufenprox, flumethrin, fluvalinate, fubfenprox, gamma-cyhalothrin, imiprothrin, kadethrin, lambda-cyhalothrin, metofluthrin, permethrin (cis-, trans-), phenothrin (1R-trans isomer), prallethrin, profluthrin, protrifenbute, pyresmethrin, resmethrin, RU 15525, silafluofen, tau-fluvalinate, tefluthrin, terallethrin, tetramethrin (-1R- isomer), tralomethrin, transfluthrin, ZXI 8901, pyrethrins (pyrethrum);
DDT; oxadiazines, such as for example indoxacarb.

Acetylcholine receptor agonists/antagonists, like chloronicotinyls, such as for example acetamiprid, clothianidin, dinotefuran, imidacloprid, nitenpyram, nithiazine, thiacloprid, thiamethoxam, nicotine, bensultap, cartap.

Acetylcholine receptor modulators, like Spinosynes, such as for example spinosad.

GABA controlled chloride channel antagonists, like Organochlorinee, such as for example camphechlor, chlordane, endosulfan, gamma-HCH, HCH, heptachlor, lindane, methoxychlor; Fiproles, such as for example acetoprole, ethiprole, fipronil, pyrafluprole, pyriprole, and vaniliprole.

Chloride channel activators, like Mectins, such as for example avermectin, emamectin, emamectin benzoate, ivermectin, milbemycin, latidectin, lepimectin, selamectin, doramectin, eprinomectin, and moxidectin.

Juvenile hormone mimetics, like for example diofenolan, epofenonane, fenoxycarb, hydroprene, kinoprene, methoprene, pyriproxifen, and triprene.

Latrophilin receptor agonists, like depsipeptides, preferably cyclic depsipetides, in particular 24-membered cyclic depsipeptides, for example emodepside.

Ecdysone agonists/disruptors, like diacylhydrazines, such as for example chromafenozide, halofenozide, methoxyfenozide, tebufenozide.

Inhibitors of chitin biosynthesis, like Benzoylureas, such as for example bistrifluron, chlofluazuron, diflubenzuron, fluazuron, flucycloxuron, flufenoxuron, hexaflumuron, lufenuron, novaluron, noviflumuron, penfluron, teflubenzuron, triflumuron; buprofezin; cyromazine.

Inhibitors of oxidative phosphorylation, ATP disruptors such as diafenthiuron; organotin compounds, such as for example azocyclotin, cyhexatin, fenbutatin-oxide.

Decouplers of oxidative phosphorylation by interruption of H-proton gradients like pyrrole, such as for example chlorfenapyr; dinitrophenols, such as for example binapacyrl, dinobuton, dinocap, DNOC.

Site I electron transport inhibitors, like METI's, such as for example fenazaquin, fenpyroximate, pyrimidifen, pyridaben, tebufenpyrad, tolfenpyrad; hydramethylnon; dicofol.

Site II electron transport inhibitors, like rotenones.

Site III electron transport inhibitors,like acequinocyl, fluacrypyrim.

Microbial disruptors of insect intestinal membrane such Bacillus thuringiensis strains.

Inhibitors of fat synthesis, like tetronic acids, such as for example spirodiclofen, spiromesifen; tetramic acids, such as for example spirotetramat (CAS-Reg.-No.: 203313-25-1) and 3-(2,5-dimethylphenyl)-8-methoxy-2-oxo-1-azaspiro[4.5]dec-3-en-4-yl ethyl carbonate (alias: carbonic acid, 3-(2,5-dimethylphenyl)-8-methoxy-2-oxo-1-azaspiro[4.5]dec-3-en-4-yl ethyl ester, CAS-Reg.-No.: 382608-10-8); carboxamides, such as for example flonicamid.

Octopaminergic agonists, such as for example amitraz.

Inhibitor of magnesium-stimulated ATPase, like propargite benzoic acid dicarboxamides, such as for example flubendiamide; Nereistoxin analogous, such as for example thiocyclam hydrogen oxalate, thiosultap-sodium.

Biologicals, hormones or pheromones like azadirachtin, Bacillus spec., Beauveria spec., codlemone, Metarrhizium spec., Paecilomyces spec., thuringiensin, Verticillium spec.

Active compounds with unknown or non-specific mode of action, like fumigants, such as for example aluminium phosphide, methyl bromide, sulphuryl fluoride; feeding inhibitors, such as for example cryolite, flonicamid, pymetrozine; mite growth inhibitors, such as for example clofentezine, etoxazole, hexythiazox; amidoflumet, benclothiaz, benzoximate, bifenazate, bromopropylate, buprofezin, quinomethionate, chlordimeform, chlorobenzilate, chloropicrin, clothiazoben, cycloprene, cyflumetofen, dicyclanil, fenoxacrim, fentrifanil, flubenzimine, flufenerim, flutenzin, gossyplure, hydramethylnone, japonilure, metoxadiazone, petroleum, piperonyl butoxide, potassium oleate, pyridalyl, sulfluramid, tetradifon, tetrasul, triarathene, and verbutin.

The content or concentration of the active compounds of formula (I) of the present invention in commercially useful forms may vary widely.

In particular, the concentration of the active compounds of formula (I) of the present invention may vary from 0.0000001 to 100 % by weight, preferably from 0.00001 to 1% by weight and further preferably from 0.0001 to 0.5 % by weight.

According to the invention, the compounds of formula (I) be used in a conventional method fit to the working forms.

Application is in a manner appropriate for the use forms.

In the agricultural field, i.e. in the field of plant protection, all plants and plant parts can be treated in accordance with the invention. Plants are to be understood as meaning in the present context all plants and plant populations such as desired and undesired wild plants or crop plants (including naturally occurring crop plants). Crop plants can be plants which can be obtained by conventional plant breeding and optimization methods or by biotechnological and genetic engineering methods or by combinations of these methods, including the transgenic plants and including the plant cultivars protectable or not protectable by plant breeders' rights. Plant parts are to be understood as meaning all parts and organs of plants above and below the ground, such as shoot, leaf, flower and root, examples which may be mentioned being leaves, needles, stalks, stems, flowers, fruit bodies, fruits, seeds, roots, tubers and rhizomes. The plant parts also include harvested material, and vegetative and generative propagation material, for example cuttings, tubers, rhizomes, offshoots and seeds.

Treatment according to the invention of the plants and plant parts with the compound according to the invention is carried out directly or by allowing the compound or to act on the surroundings, habitat or storage space by the customary treatment methods, for example by watering (drenching), drip irrigation, spraying, vaporizing, atomizing, broadcasting, dusting, foaming, spreading-on, and as a powder for dry seed treatment, a solution for seed treatment, a water-soluble powder for seed treatment, a water-soluble powder for slurry treatment, or by encrusting, and in the case of propagation material, in particular in the case of seeds, moreover by dry treatments, slurry treatments, liquid treatments, by one- or multi-layer coating.. It is furthermore possible to apply the active compounds by the ultra-low volume method, or to inject the active compound preparation or the active compound itself into the soil.

The compounds according to the invention are particularly suitable for treating seed. Thus, a large part of the damage to crop plants which is caused by pests occurs as early as when the seed is attacked during storage and after the seed is introduced into the soil, during and immediately after germination of the plants. This phase is particularly critical since the roots and shoots of the growing plant are particularly sensitive and even minor damage can lead to the death of the whole plant. Protecting the seed and the germinating plant by the use of suitable compositions comprising the compound according to the invention is therefore of particularly great interest.

The control of pests by treating the seeds of plants has been known for a long time and is subject-matter of continuous improvements. However, the treatment of seed frequently entails a series of problems which cannot always be solved in a satisfactory manner. Thus, it is desirable to develop methods for protecting the seed and the germinating plant which dispense with the additional application of crop protection agents after sowing or after the emergence of the plants or where additional applications are at least reduced. It is furthermore desirable to optimize the amount of active compound employed in such a way as to provide maximum protection for the seed and the germinating plant from attack by pests, but without damaging the plant itself by the active compound employed. In particular, methods for the treatment of seed should also take into consideration the intrinsic insecticidal properties of transgenic plants in order to achieve optimum protection of the seed and the germinating plant with a minimum of crop protection agents being employed.

In the agricultural field, the dose of active compound/ application rate usually applied in the method of treatment according to the invention is for foliar treatments: from 0.1 to 10,000 g/ha, preferably from 10 to 1,000 g/ha, more preferably from 50 to 300g/ha; in case of drench or drip application, the dose can be reduced; for seed treatment: from 2 to 200 g per 100 kilogram of seed, preferably from 3 to 150 g per 100 kilogram of seed; and for soil treatment: from 0.1 to 10,000 g/ha, preferably from 1 to 5,000 g/ha.

As already mentioned before, it is possible to treat all plants and their parts according to the invention. In a preferred embodiment, wild plant species and plant cultivars, or those obtained by conventional biological breeding methods, such as crossing or protoplast fusion, and parts thereof, are treated. In a further preferred embodiment, transgenic plants and plant cultivars obtained by genetic engineering methods, if appropriate in combination with conventional methods (Genetically Modified Organisms), and parts thereof are treated. The terms "parts", "parts of plants" and "plant parts" have been explained above.

Particularly preferably, plants of the plant cultivars which are in each case commercially available or in use are treated according to the invention. Plant cultivars are to be understood as meaning plants having novel properties ("traits") which have been obtained by conventional breeding, by mutagenesis or by recombinant DNA techniques. These can be cultivars, bio- or genotypes.

The transgenic plants or plant cultivars (obtained by genetic engineering) which are preferably to be treated according to the invention include all plants which, by virtue of the genetic modification, received genetic material which imparted particularly advantageous, useful traits to these plants. Examples of such traits are better plant growth, increased tolerance to high or low temperatures, increased tolerance to drought or to water or soil salt content, increased flowering performance, easier harvesting, accelerated maturation, higher harvest yields, higher quality and/or a higher nutritional value of the harvested products, better storage stability and/or processability of the harvested products. Further and particularly emphasized examples of such traits are a better defence of the plants against animal and microbial pests, such as against insects, mites, phytopathogenic fungi, bacteria and/or viruses, and also increased tolerance of the plants to certain herbicidally active compounds. Examples of transgenic plants which may be mentioned are the important crop plants, such as cereals (wheat, rice), maize, soya beans, potatoes, sugar beet, tomatoes, peas and other vegetable varieties, cotton, tobacco, oilseed rape and also fruit plants (with the fruits apples, pears, citrus fruits and grapes), and particular emphasis is given to maize, soya beans, potatoes, cotton, tobacco and oilseed rape. Traits that are emphasized are in particular increased defence of the plants against insects, arachnids, nematodes and slugs and snails by virtue of toxins formed in the plants, in particular those formed in the plants by the genetic material from Bacillus thuringiensis (for example by the genes CryIA(a), CryIA(b), CryIA(c), CryIIA, CryIIIA, CryIIIB2, Cry9c, Cry2Ab, Cry3Bb and CryIF and also combinations thereof) (referred to hereinbelow as "Bt plants"). Traits that are also particularly emphasized are the increased defence of the plants against fungi, bacteria and viruses by systemic acquired resistance (SAR), systemin, phytoalexins, elicitors and resistance genes and correspondingly expressed proteins and toxins. Traits that are furthermore particularly emphasized are the increased tolerance of the plants to certain herbicidally active compounds, for example imidazolinones, sulphonylureas, glyphosate or phosphinotricin (for example the "PAT" gene). The genes which impart the desired traits in question can also be present in combination with one another in the transgenic plants. Examples of "Bt plants" which may be mentioned are maize varieties, cotton varieties, soya bean varieties and potato varieties which are sold under the trade names YIELD GARD® (for example maize, cotton, soya beans), KnockOut® (for example maize), StarLink® (for example maize), Bollgard® (cotton), Nucotn® (cotton) and NewLeaf® (potato). Examples of herbicide-tolerant plants which may be mentioned are maize varieties, cotton varieties and soya bean varieties which are sold under the trade names Roundup Ready® (tolerance to glyphosate, for example maize, cotton, soya bean), Liberty Link® (tolerance to phosphinotricin, for example oilseed rape), IMI® (tolerance to imidazolinones) and STS® (tolerance to sulphonylureas, for example maize). Herbicide-resistant plants (plants bred in a conventional manner for herbicide tolerance) which may be mentioned include the varieties sold under the name Clearfield® (for example maize). Of course, these statements also apply to plant cultivars having these genetic traits or genetic traits still to be developed, which plant cultivars will be developed and/or marketed in the future.

The plants listed can be treated in a particularly advantageous manner with the active compound according to the invention. The preferred ranges stated above for the active compound also apply to the treatment of these plants.

When the active compounds of the present invention are used in the hygiene sector, particularly are used against hygiene pests or pests in stored substances, they have stability against alkali on calciphilous materials and produces excellent residual effects on woods and soils.

Next, the present invention will be more specifically explained by way of examples, but the present invention is not intended to be limited to these examples.

### Synthetic Example 1 (synthesis of starting material).

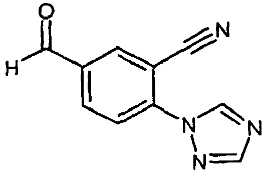

2-fluoro-5-formylbenzonitrile (1.0 g, 6.71 mmol) and 1H-1,2,4-triazole (0.56 g, 8.05 mmol) were dissolved in DMF. Potassium carbonate (1.1 g, 8.05 mmol) was added to the solution, which was stirred at 120°C for 6 hours. The temperature of this reaction solution was returned to room temperature, and water and ethyl acetate were added to the solution to separate the organic layer and the aqueous layer was extracted with ethyl acetate. The organic layers were combined, washed with water and dried over magnesium sulfate anhydride. After the desiccating agent was separated by filtration, the filtrate was concentrated under reduced pressure. The obtained crude product was purified by silica gel column chromatography (hexane/ethyl acetate) to obtain 0.60 g of 5-formyl-2-(1H-1,2,4-triazol-1-yl)benzonitrile (m.p.: 134-141 1°C, yield: 43%),

### Synthetic Example 2 (synthesis of starting material)

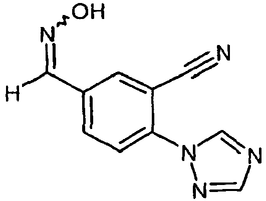

5-formyl-2-(1H-1,2,4-triazol-1-yl)benzonitrile (1.58 g, 7.97 mmol) and hydroxylamine hydrochloride (0.67 g, 9.57 mmol) were dissolved in a THF-water (4 : 1) mixed solvent. Sodium acetate (0.92 g, 11.2 mmol) was added to the solution, which was stirred at room temperature for 4 hours. After the reaction was completed, water and ethyl acetate were added to the reaction solution to separate the organic layer and the aqueous layer was extracted with ethyl acetate. The organic layers were combined, washed with water and dried over magnesium sulfate anhydride. After the desiccating agent was separated by filtration, the filtrate was concentrated under reduced pressure' to obtain 1.56 g of 5-[(hydroxyimino)methyl]-2-(1H-1,2,4-triazol-1-yl)benzonitrile (m.p.: 198-200°C, yield: 87%).

### Synthetic Example 3

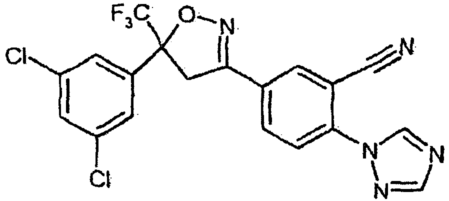

N-chlorosuccinimide (0.41 g, 3.04 mmol) was added to a mixture obtained by dissolving 5-[(hydroxyimino)methyl]-2-(1H-1,2,4-triazol-1-yl)benzonitrile (0.59 g, 2.77 mmol) in DMF and the mixture was stirred for 2 hours. 1,3-dichloro-5-[1-(trifluoromethyl)vinyl)benzene (0.82 g, 3.04 mmol) was further added to the mixture. Triethylamine (0.31 g, 3.29 mmol) dissolved in DMF was added dropwise to the above mixture under ice-cooling. After the addition was completed, the resulting mixture was stirred for 2 hours at the same temperature and then stirred for more 4 hours after the temperature of the mixture was returned to room temperature. After the reaction was completed, water and ethyl acetate were added to the reaction solution to separate the organic layer and the aqueous layer was extracted with ethyl acetate. The organic layers were combined, washed with water and dried over magnesium sulfate anhydride. After the desiccating agent was separated by filtration, the filtrate was concentrated under reduced pressure. The obtained crude product was purified by silica gel column chromatography (hexane/ethyl acetate) to obtain 0.51 g of 5-[5-(3,5-dichlorophenyl)-5-(trifluoromethyl)-4,5-dihydroisoxazol-3-yl)-2-(1H-1,2,4-triazol-1-yl)benzonitrile (m.p.: 118-125°C, yield: 39%).

### Synthetic Example 4 (illustrative example)

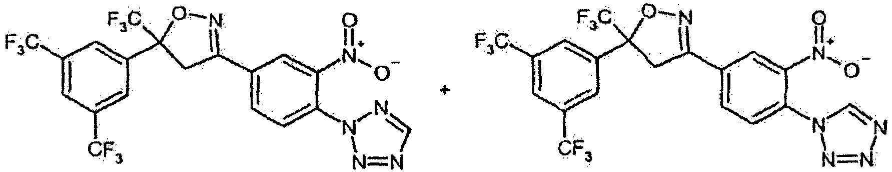

5-[3,5-bis(trifluoromethyl)phenyl]-3-(4-fluoro-3-nitrophenyl)-5-(trifluoromethyl)-4,5-dihydroisoxazole (0.40 g, 0.82 mmol) and 1H-tet-azole (0.09 g, 1.22 mmol) were dissolved in DMF. Potassium carbonate (0.17 g, 1.25 mmol) was added to the solution, which was stirred at 60°C for 6 hours. The temperature of this reaction solution was returned to room temperature, and water and ethyl acetate were added to the solution to separate the organic layer and the aqueous layer was extracted with ethyl acetate. The organic layers were combined, washed with water and dried over magnesium sulfate anhydride. After the desiccating agent was separated by filtration, the filtrate was concentrated under reduced pressure. The obtained crude product was purified by silica gel column chromatography (hexane/ethyl acetate) to obtain 0.06 g of 1-(4-{5-[3,5-bis(trifluoromethyl)phenyl]-5-(trifluoromethyl)-4,5-dihydroisoxazol-3-yl}-2-nitrophenyl)-2H-tetrazole (m.p.: 147-149°C, yield: 13%) and 0.28 g of 1-(4-{5-[3,5-bis(trifluoromethyl)phenyl]-5-(trifluoromethyl)-4,5-dihydroisoxazol-3-yl}-2-nitrophenyl)-1H-tetrazole (m.p.: 173-175°C, yield: 60%).

### Synthetic Example 5 (illustrative example)

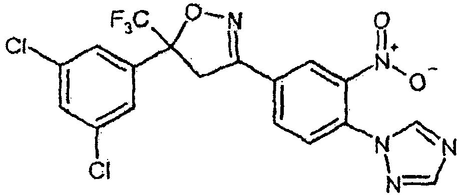

3-(4-fluoro-3-nitrophenyl)-5-(3,5-dichlorophenyl)-5-(trifluoromethyl)-4,5-dihydroisoxazole (0.6 g, 1.42 mmol) and 1H-1,2,4-triazole (0.12 g, 1.70 mmol) were dissolved in DMF. Potassium carbonate (0.24 g, 1.70 mmol) was added to the solution, which was stirred at 60°C for 6 hours. The temperature of this reaction solution was returned to room temperature, and water and ethyl acetate were added to the solution to separate the organic layer and the aqueous layer was extracted with ethyl acetate. The organic layers were combined, washed with water and dried over magnesium sulfate anhydride. After the desiccating agent was separated by filtration, the filtrate was concentrated under reduced pressure. The obtained crude product was purified by silica gel column chromatography (hexane/ethyl acetate) to obtain 0.66 g of 1-{4-[5-[3,5-dichlorophenyl]-5-(trifluoromethyl)-4,5-dihydroisoxazol-3-yl]-2-nitrophenyl}-1H-1,2,4-triazole (m.p.: 64-72°C, yield: 94%).

### Synthetic Example 6

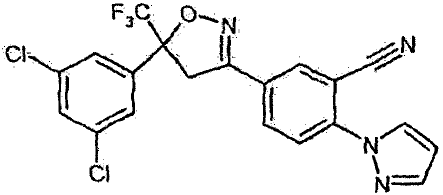

1H-pyrazole (0.06 g, 0.89 mmol) was dissolved in DMF. Sodium hydride (60%, 0.06 g, 0.89 mmol) was added to the solution under ice-cooling, and then the temperature of the solution was returned to room temperature. The solution was stirred for 0.5 hours and was ice-cooled again. 5-[5-(3,5-dichlorophenyl)-5-(trifluoromethyl)-4,5-dihydroisoxazol-3-yl]-2-fluorobenzonitrile (0.30 g, 0.74 mmol) dissolved in DMF was added dropwise to the mixture. After the addition was completed, the temperature of this reaction solution was returned to room temperature, and the solution was stirred for 3 hours. After the reaction was completed, water and ethyl acetate were added to the solution to separate the organic layer and the aqueous layer was extracted with ethyl acetate. The organic layers were combined, washed with water and dried over magnesium sulfate anhydride. After the desiccating agent was separated by filtration, the filtrate was concentrated under reduced pressure. The obtained crude product was purified by silica gel column chromatography (hexane/ethyl acetate) to obtain 0.2 g of 5-[5-(3,5-dichlorophenyl)-5-(trifluoromethyl)-4,5-dihydroisoxazol-3-yl]-2-(1H-pyrazol-1-yl)benzonitrile (m.p.: 169-176°C, yield: 57%).

### Synthetic Example 7

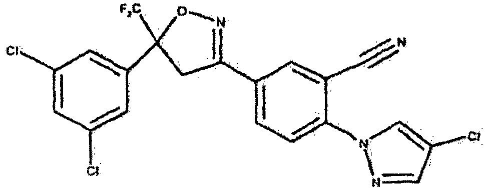

5-[5-(3,5-dichlorophenyl)-5-(trifluoromethyl)-4,5-dihydroisoxazol-3-yl]-2-(1H-pyrazol-1-yl)benzonitrile (0.43 g, 0.95 mmol) was dissolved in DMF. N-chlorosuccinimide (0.14 g, 1.05 mmol) was added to this solution, which was then stirred at room temperature for 2 hours and at 80°C for 2 hours. After the reaction was completed, water and ethyl acetate were added to the solution to separate the organic layer and the aqueous layer was extracted with ethyl acetate. The organic layers were combined, washed with water and dried over magnesium sulfate anhydride. After the desiccating agent was separated by filtration, the filtrate was concentrated under reduced pressure. The obtained crude product was purified by silica gel column chromatography (hexane/ethyl acetate) to obtain 0.2 g of 2-(4-chloro-1H-pyrazol-1-yl)-5-[5-(3,5-dichlorophenyl)-5-(trifluoromethyl)-4,5-dihydroisoxazol-3-yl]benzonitrile (m.p.: 190-191°C, yield: 41%).

### Synthetic Example 8 (illustrative exampe)

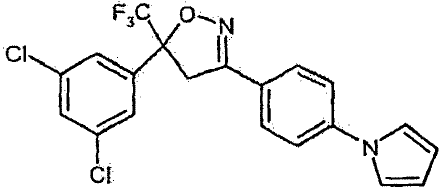

4-[5-(3,5-dichlorophenyl)-5-(trifluoromethyl)-4,5-dihydroisoxazol-3-yl]aniline (0.30 g, 0.80 mmol) and 2,5-dimethoxytetrahydrofuran (0.26 g, 2.00 mmol) were dissolved in acetic acid. The solution was refluxed under heating for 0.5 hours. The temperature of the reaction solution was returned to room temperature and water and ethyl acetate were added to the solution to separate the organic layer and the aqueous layer was extracted with ethyl acetate. The organic layers were combined, washed with water and dried over magnesium sulfate anhydride. After the desiccating agent was separated by filtration, the filtrate was concentrated under reduced pressure. The obtained crude product was purified by silica gel column chromatography (hexane/ethyl acetate) to obtain 0.22 g of 5-(3,5-dichlorophenyl)-3-[4-(1H-pyrrol-1-yl)phenyl]-5-(trifluoromethyl)-4,5-dihydroisoxazole (m.p.: 206-208°C, yield: 61 %).

### Synthetic Example 9 (illustrative example)

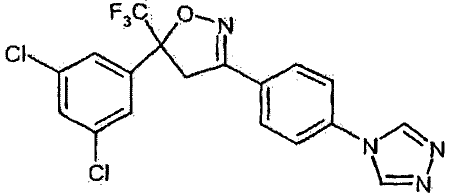

4-[5-(3,5-dichlorophenyl)-5-(trifluoromethyl)-4,5-dihydroisoxazol-3-yl]aniline (0.30 g, 0.80 mmol) and 1, 2-diformylhydrazine (0.18 g, 2.00 mmol) were suspended in pyridine. Triethylamine (0.57 g, 5.6 mmol) and trimethylchlorosilane (1.30 g, 12. 0 mmol) were sequentially added to the suspension liquid under ice-cooling. Thereafter, the liquid was refluxed under heating for 4 hours. The temperature of the reaction solution was returned to room temperature and then water was added to the reaction solution to obtain a precipitate. The precipitate was washed with a small amount of ethyl acetate and dried to obtain 0.14 g of 4-{4-[5-(3,5-dichlorophenyl)-5-(trifluoromethyl)-4,5-dihydroisoxazol-3-yl]phenyl}-4H-1,3,4-triazole (m.p. > 250°C, yield: 39%).

### Synthetic Example 10 (illustrative example)

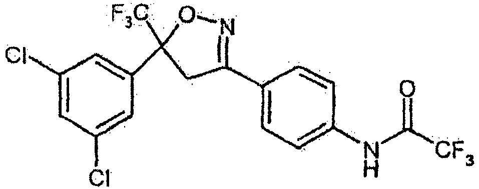

4-[5-(3,5-dichlorophenyl)-5-(trifluoromethyl)-4,5-dihydroisoxazol-3-yl]aniline (1.25 g, 3.33 mmol) and triethylamine (0.40 g, 3.95 mmol) were dissolved in dichloromethane. A dichloromethane solution of trifluoroacetic acid anhydride (0.80 g, 3.81 mmol) was added to the solution under ice-cooling and the mixture was stirred at room temperature for one hour. After the reaction was completed, the reaction solution was washed with water and dried over magnesium sulfate anhydride. After the desiccating agent was separated by filtration, the filtrate was concentrated under reduced pressure to obtain 1.55 g of N-{4-[5-(3,5-dichlorophenyl)-5-(trifluoromethyl)-4,5-dihydroisoxazol-3-yl]phenyl}-2,2,2-trifluoroacetamide (m.p.: 45-52°C, yield: 99%).

### Synthetic Example 11 (illustrative example)

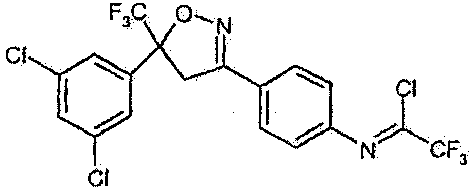

N- {4-[5-(3,5-dichlorophenyl)-5-(trifluoromethyl)-4,5-dihydroisoxazol-3-yl]phenyl} -2,2,2-trifluoroacetamide (1.30 g, 2.76 mmol) and triphenylphosphine (1.00 g, 3.81 mmol) were dissolved in dichloromethane. Carbon tetrachloride (0.60 g, 3.90 mmol) was added to the solution at 30°C and the resulting solution was refluxed under heating for 5 hours. After the reaction was completed, a crude product obtained by concentrating the reaction solution under reduced pressure was purified by silica gel column chromatography (hexane/ethyl acetate) to obtain 1.20 g of N-{4-[5-(3,5-dichlorophenyl)-5-(trifluoromethyl)-4,5-dihydroisoxazol-3-yl]phenyl}-2,2,2-trifluoroethanimidoyl chloride (yield: 89%).

### Synthetic Example 12 (illustrative example)

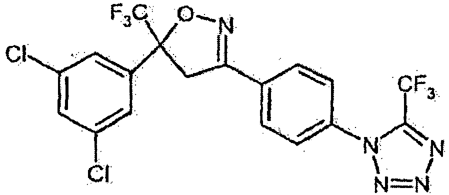

N-{4-[5-(3,5-dichlorophenyl)-5-(trifluoromethyl)-4,5-dihydroisoxazom-3-yl]phenyl }-2,2,2-trifluoroethanimidoyl chloride (0.125 g, 0.255 mmol) was dissolved in acetonitrile. Sodium azide (0.05 g, 0.769 mmol) was added to the solution, which was then stirred at room temperature for 15 hours. After the reaction was completed, water and ethyl acetate were added to the solution to separate the organic layer and the aqueous layer was extracted with ethyl acetate. The organic layer was dried over magnesium sulfate anhydride. After the desiccating agent was separated by filtration, the filtrate was concentrated under reduced pressure. The obtained crude product was purified by silica gel column chromatography (hexane/ethyl acetate) to obtain 0.10 g of 1-{4-[5-(3,5-dichlorophenyl)-5-(trifluoromethyl)-4,5-dihydroisoxazol-3-yl]phenyl}-5-(trifluoromethyl)-1H-tetrazole (m.p.: 147-151°C, yield: 79%).

### Synthetic Example 13 (illustrative example)

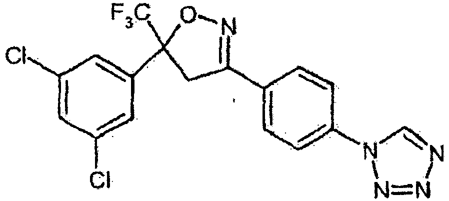

4-[5-(3,5-dichlorophenyl)-5-(trifluoromethyl)-4,5-dihydroisoxazol-3-yl]aniline (0.40 g, 1.07 mmol) and ethyl orthoformate (0.30 g, 2.02 mmol) were dissolved in acetic acid. Sodium azide (0.10 g, 1.54 mmol) was added to the solution, which was then refluxed under heating for 5 hours. After the reaction was completed, water and ethyl acetate were added to the solution to separate the organic layer and the aqueous layer was extracted with ethyl acetate. The organic layer was dried over magnesium sulfate anhydride. After the desiccating agent was separated by filtration, the filtrate was concentrated under reduced pressure. The obtained crude product was purified by silica gel column chromatography (hexane/ethyl acetate) to obtain 0.25 g of 1-{4-[5-(3,5-dichlorophenyl)-5-(trifluoromethyl)-4,5-dihydroisoxazol-3-yl]phenyl}-1H-tetrazole (m.p.: 198-199°C (decomposed), yield: 55%).

### Synthetic Example 14 (illustrative example)

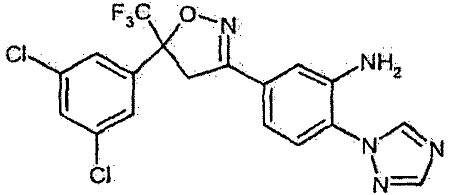

1- {4-[5-(3,5-dichlorophenyl)-5-(trifluoromethyl)-4,5-dihydroisoxazol-3-yl]-2-nitrophenyl}-1H-1,2,4-triazole (0.53 g, 1.12 mmol) and stannous chloride dihydrate (1.01 g, 4.49 mmol) were suspended in ethanol. Moreover, a catalytic amount of concentrated hydrochloric acid was added to the solution. The reaction solution was heated at 60°C for 4 hours. After the reaction was completed, the temperature of the solution was returned to room temperature. Water and ethyl acetate were added to the solution, which was then neutralized using potassium carbonate with vigorously stirring. The suspension was passed through Celite. The organic layer was separated and the aqueous layer was extracted with ethyl acetate. The organic layers were combined, washed with water and dried over magnesium sulfate anhydride. After the desiccating agent was separated by filtration, the filtrate was concentrated under reduced pressure. The obtained crude product was purified by silica gel column chromatography (hexane/ethyl acetate) to obtain 0.38 g of 5-[5-(3,5-dichlorophenyl)-5-(trifluoromethyl)-4,5-dihydroisoxazol-3-yl]-2-(1H-1,2,4-triazol-1-yl)aniline (m.p.: 244-246°C, yield: 73%).

### Synthetic Example 15 (illustrative example)

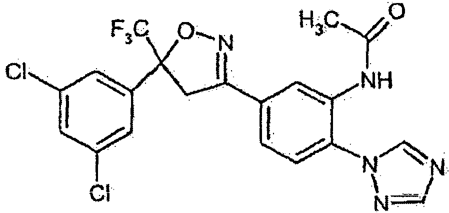

5-[5-(3,5-dichlorophenyl)-5-(trifluoromethyl)4,5-dihydroisoxazol-3-yl]-2-(1H-1,2,4-triazol-1-yl)aniline (0.29 g, 0.66 mmol) and pyridine (0.08 g, 0.98 mmol) were dissolved in THF. Acetyl chloride (0.05 g, 0.69 mmol) was added to this solution at room temperature and the solution was stirred for I hour. After the reaction was completed, water and ethyl acetate were added to the solution. The organic layer was separated and the aqueous layer was extracted with ethyl acetate. The organic layers were combined, washed with water and dried over magnesium sulfate anhydride. After the desiccating agent was separated by filtration, the filtrate was concentrated under reduced pressure. The obtained crude product was purified by silica gel column chromatography (hexane/ethyl acetate) to obtain 0.17 g of N-{5-[5-(3,5-dichlorophenyl)-5-(trifluoromethyl)-4,5-dihydroisoxazol-3-yl]-2-(1H-1,2,4-triazol-1-yl)phenyl}acetamide (m.p.: 230-233°C, yield: 51%).

The compounds of the formula (I), according to the present invention obtained in the same way as the synthesis of the starting materials and the synthetic examples for synthesizing the final products, are shown in Table 1, and the specific examples of intermediates are shown in Tables 2 to 4.

Among the above synthetic examples, the compounds corresponding to the final products are shown in Table 1.

In the following tables, Me stands for methyl, Et stands for ethyl, Pr^{cyclo} stands for cyclopropyl and Ph stands for phenyl.

**Table 1 (compounds according to the invention)**

| | | | | | | | |
|---|---|---|---|---|---|---|---|
| 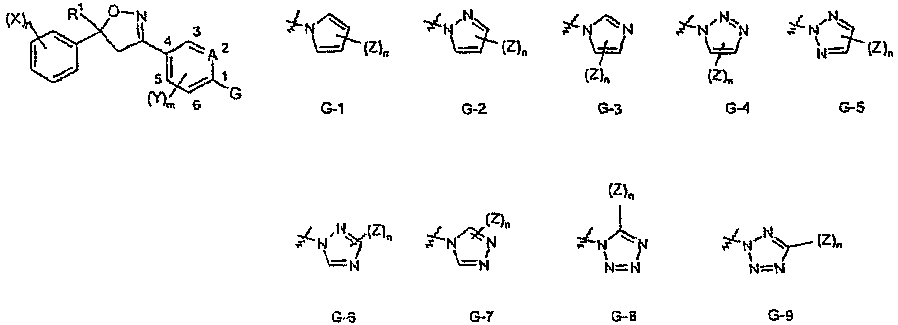 | | | | | | | |

| | (X)ₗ | R¹ | A | (Y)ₘ | G | (Z)ₙ | melting point [°C] / refractive index |
|---|---|---|---|---|---|---|---|
| **13** | 3,5-Cl₂ | CF₃ | C | 2-CN | G-2 | — | 169-176 |
| **14** | 3,5-Cl₂ | CF₃ | C | 2-CN | G-2 | 4-Me | |
| **15** | 3,5-Cl₂ | CF₃ | C | 2-CN | G-2 | 4-CF₃ | |
| **16** | 3,5-Cl₂ | CF₃ | C | 2-CN | G-2 | 4-F | 174-175 |
| **17** | 3,5-Cl₂ | CF₃ | C | 2-CN | G-2 | 4-Cl | 190-191 |
| **18** | 3,5-Cl₂ | CF₃ | C | 2-CN | G-2 | 4-Br | 168-170 |
| **19** | 3,5-Cl₂ | CF₃ | C | 2-CN | G-2 | 4-I | 68-78 |
| **20** | 3,5-Cl₂ | CF₃ | C | 2-CN | G-2 | 4-CN | |
| **21** | 3,5-Cl₂ | CF₃ | C | 2-CN | G-2 | 4-NO₂ | 101-105 |
| **33** | 3-CF₃ | CF₃ | C | 2-CN | G-2 | - | |
| **34** | 3-CF₃ | CF₃ | C | 2-CN | G-2 | 4-Cl | |
| **35** | 3-CF₃ | CF₃ | C | 2-CN | G-2 | 4-Br | |
| **36** | 3-CF₃ | CF₃ | C | 2-CN | G-2 | 4-I | |
| **37** | 3-CF₃ | CF₃ | C | 2-CN | G-2 | 4-CN | |
| **38** | 3-CF₃ | CF₃ | C | 2-CN | G-2 | 4-NO₂ | 61-68 |
| **42** | 3,5-(CF₃)₂ | CF₃ | C | 2-CN | G-2 | - | |
| **43** | 3,5-(CF₃)₂ | CF₃ | C | 2*-*CN | G-2 | 4-Cl | |
| **44** | 3,5-(CF₃)₂ | CF₃ | C | 2-CN | G-2 | 4-Br | |
| **45** | 3,5-(CF₃)₂ | CF₃ | C | 2-CN | G-2 | 4-1 | |
| **46** | 3,5-(CF₃)₂ | CF₃ | C | 2-CN | G-2 | 4-CN | |
| **47** | 3,5-(CF₃)₂ | CF₃ | C | 2-CN | G-2 | 4-NO₂ | NMR |
| **51** | 3,5-Cl₂ | CF₃ | C | 2-CN | G-3 | - | |
| **55** | 3-CF₃ | CF₃ | C | 2-CN | G-3 | - | |
| **59** | 3,5-(CF₃)₂ | CF₃ | C | 2-CN | G-3 | - | |
| **63** | 3,5-Cl₂ | CF₃ | C | 2-CN | G-4 | - | |
| **66** | 3-CF₃ | CF₃ | C | 2-CN | G-4 | - | |
| **68** | 3,5-(CF₃)₂ | CF₃ | C | 2-CN | G-4 | - | |
| **71** | 3.5-Cl₂ | CF₃ | C | 2-CN | G-5 | - | |
| **74** | 3-CF₃ | CF₃ | C | 2-CN | G-5 | - | |
| **76** | 3,5-(CF₃)₂ | CF₃ | C | 2-CN | G-5 | - | |
| **80** | | CF₃ | C | 2-CN | G-6 | - | NMR |
| **84** | 3,5-F₂ | CF₃ | C | 2-CN | G-6 | - | |
| **89** | 3-Cl | CF₃ | C | 2-CN | G-6 | - | 49-58 |
| **101** | | 3,5-Cl₂ | CF₃ C | 2-CN | G-6 | - | 18-125 |
| **102** | | 3,5-Cl₂ | CF₂CF₃ C | 2-CN | G-6 | - | |
| **118** | | 3-Br | CF₃ C | 2-CN | G-6 | - | |
| **121** | | 3,5-Br₂ | CF₃ C | 2-CN | G-6 | - | |
| **126** | 3-CF₃ | CF₃ | C | 2-CN | G-6 | - | 1.5505 (24°C) |
| **135** | 3,5-(CF₃)₂ | CF₃ | C | 2-CN | G-6 | - | NMR |
| **143** | 3,5-Cl₂ | CF₃ | C | 2-CN | G-7 | - | |
| **146** | 3-Cl | CF₃ | C | 2-CN | G-8 | - | |
| **153** | 3,5-Cl₂ | CF₃ | C | 2-CN | G-8 | - | 80-89 |
| **160** | 3-CF₃ | CF₃ | C | 2-CN | G-8 | - | 109-117 |
| **165** | 3,5-(CF₃)₂ | CF₃ | C | 2-CN | G-8 | - | 145-151 |
| **171** | 3,5-Cl₂ | CF₃ | C | 2-CN | G-9 | - | 167-169 |
| **175** | 3-CF₃ | CF₃ | C | 2-CN | G-9 | - | |
| **177** | 3,5-(CF₃)₂ | CF₃ | C | 2-CN | G-9 | - | |
| **180** | 3,4-Cl₂ | CF₃ | C | 2-CN | G-6 | - | 57-60 |
| **181** | 3,5-Cl₂ | CF₃ | C | 2-CN | G-2 | 4-NH₂ | 218-222 |
| **182** | 3,5-Cl₂ | CF₃ | C | 2-CN | G-6 | 3-NO₂ | |
| **183** | 3-CF₃ | CF₃ | C | 2-CN | G-6 | 3-NO₂ | |
| **184** | 3,5-(CF₃)₂ | CF₃ | C | 2-CN | G-6 | 3-NO₂ | |

| | | | | | | | |
|---|---|---|---|---|---|---|---|
| NMR analysis | | | | | | | |

In the following tables, Me stands for methyl, Et stands for ethyl, Pr^{cyclo} stands for cyclopropyl and Ph stands for phenyl.

**Table 1a (illustrative examples)**

| | | | | | | | |
|---|---|---|---|---|---|---|---|
| 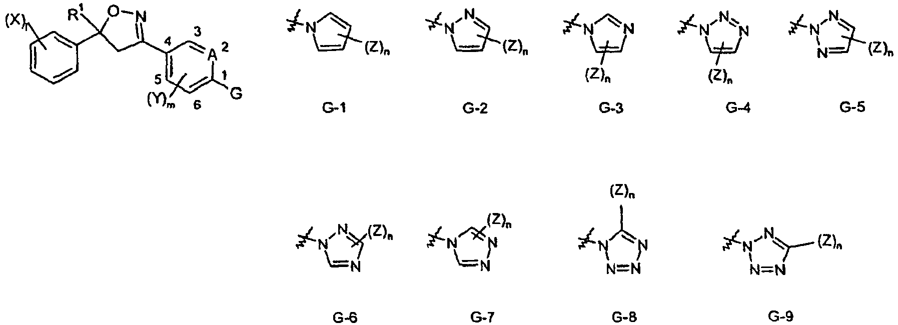 | | | | | | | |

| | (X)₁ | R¹ | A | (Y)ₘ | G | (Z)ₙ | melting point [°C] / refractive index |
|---|---|---|---|---|---|---|---|
| **1** | 3,5-Cl₂ | CF₃ | C | - | G-1 | - | 206-208 |
| **2** | 3,5-Cl₂ | CF₃ | C | - | G-2 | - | 159-164 |
| **3** | 3,5-Cl₂ | CF₃ | C | - | G-2 | 3-CF₃ | 112-117 |
| **4** | 3,5-Cl₂ | CF₃ | C | - | G-2 | 4-Cl | 197-199 |
| **5** | 3,5-Cl₂ | CF₃ | C | - | G-2 | 4-Br | 189-192 |
| **6** | 3,5-Cl₂ | CF₃ | C | - | G-2 | 4-I | 184-185 |
| **7** | 3,5-Cl₂ | CF₃ | C | - | G-2 | 4-CN | 205-206 |
| **8** | 3,5-Cl₂ | CF₃ | C | 2-Cl | G-2 | - | 43-51 |
| **9** | 3,5-Cl₂ | CF₃ | C | 2-Br | G-2 | - | |
| **10** | 3,5-Cl₂ | CF₃ | C | 2-Br | G-2 | 4-Cl | |
| **11** | 3,5-Cl₂ | CF₃ | C | 2-Br | G-2 | 4-Br | |
| **12** | 3,5-Cl₂ | CF₃ | C | 2-Br | G-2 | 4-NO₂ | 61-64 |
| **22** | 3,5-Cl₂ | CF₃ | C | 2-NO₂ | G-2 | 3-CF₃ | 50-56 |
| **23** | 3,5-Cl₂ | CF₃ | C | 2-NO₂ | G-2 | - | 184-185 |
| **24** | 3,5-Cl₂ | CF₃ | C | 2-NO₂ | G-2 | 4-Me | 66-78 |
| **25** | 3,5-Cl₂ | CF₃ | C | 2-NO₂ | G-2 | 4-F | 177-180 |
| **26** | 3,5-Cl₂ | CF₃ | C | 2-NO₂ | G-2 | 4-Cl | 67-74 |
| **27** | 3,5-Cl₂ | CF₃ | C | 2-NO₂ | G-2 | 4-Br | 68-71 |
| **28** | 3,5-Cl₂ | CF₃ | C | 2-NO₂ | G-2 | 4-I | 76-78 |
| **29** | 3,5-Cl₂ | CF₃ | C | 2-NO₂ | G-2 | 4-CF3 | 62-67 |
| **30** | 3-CF₃ | CF₃ | C | 2-Br | G-2 | - | |
| **31** | 3-CF₃ | CF₃ | C | 2-Br | G-2 | 4-Cl | |
| **32** | 3-CF₃ | CF₃ | C | 2-Br | G-2 | 4-Br | |
| **39** | 3,5-(CF₃)₂ | CF₃ | C | 2-Br | G-2 | - | |
| **40** | 3,5-(CF₃)₂ | CF₃ | C | 2-Br | G-2 | 4-Cl | |
| **41** | 3,5-(CF₃)₂ | CF₃ | C | 2-Br | G-2 | 4-Br | |
| **48** | 3,5-Cl₂ | CF₃ | C | - | G-3 | - | 189-196 |
| **49** | 3,5-Cl₂ | CF₃ | C | 2-Cl | G-3 | - | |
| **50** | 3,5-Cl₂ | CF₃ | C | 2-Br | G-3 | - | |
| **52** | 3,5-Cl₂ | CF₃ | C | 2-NO₂ | G-3 | - | 115-117 |
| **53** | 3-CF₃ | CF₃ | C | 2-Cl | G-3 | - | |
| **54** | 3-CF₃ | CF₃ | C | 2-Br | G-3 | - | |
| **56** | 3-CF₃ | CF₃ | C | 2-NO₂ | G-3 | - | |
| **57** | 3,5-(CF₃)₂ | CF₃ | C | 2-Cl | G-3 | - | |
| **58** | 3,5-(CF₃)₂ | CF₃ | C | 2-Br | G-3 | - | |
| **60** | 3,5-(CF₃)₂ | CF₃ | C | 2-NO₂ | G-3 | - | |
| **61** | 3,5-Cl₂ | CF₃ | C | | G-4 | - | |
| **62** | 3,5-Cl₂ | CF₃ | C | 2-Br | G-4 | - | |
| **64** | 3,5-Cl₂ | CF₃ | C | 2-NO₂ | G-4 | - | 165-170 |
| **65** | 3-CF₃ | CF₃ | C | 2-Br | G-4 | - | |
| **67** | 3,5-(CF₃)₂ | CF₃ | C | 2-Br | G-4 | - | |
| **69** | 3,5-Cl₂ | CF₃ | C | - | G-5 | - | |
| **70** | 3,5-Cl₂ | CF₃ | C | 2-Br | G-5 | - | |
| **72** | 3,5-Cl₂ | CF₃ | C | 2-NO₂ | G-5 | - | 60-66 |
| **73** | 3-CF₃ | CF₃ | C | 2-Br | G-5 | - | |
| **75** | 3,5-(CF₃)₂ | CF₃ | C | 2-Br | G-5 | - | |
| **77** | - | CF₃ | C | - | G-6 | - | 187 |
| **78** | - | CF₃ | C | 2-Cl | G-6 | - | |
| **79** | - | CF₃ | C | 2-Br | G-6 | - | |
| **81** | 3,5-F₂ | CF₃ | C | - | G-6 | - | 167-170 |
| **82** | 3,5-F₂ | CF₃ | C | -Cl | G-6 | - | |
| **83** | 3,5-F₇ | CF₃ | C | 2-Br | G-6 | - | |
| **85** | 3,5-F₂ | CF₃ | C | 2-NO₂ | G-6 | - | 52-60 |
| **86** | 3-Cl | CF₃ | C | - | G-6 | - | 161-169 |
| **87** | 3-Cl | CF₃ | C | 2-Cl | G-6 | - | |
| **88** | 3-Cl | CF₃ | C | 2-Br | G-6 | - | |
| **90** | 3-Cl | CF₃ | C | 2-NO₂ | G-6 | - | 1.5795 (25 °C) |
| **91** | 3,5-Cl₂ | CF₃ | C | - | G-6 | - | 182-184 |
| **92** | 3,5-Cl₂ | CF₂CF₃ | C | - | G-6 | - | |
| **93** | 3,5-Cl₂ | CF₃ | C | -F | G-6 | - | 147-151 |
| **94** | 3,5-Cl₂ | CF₃ | C | 2-Cl | G-6 | - | 56-60 |
| **95** | 3,5-Cl₂ | CF₂CF₃ | C | 2-Cl | G-6 | - | |
| **96** | 3,5-Cl₂ | CF₃ | C | 3-Cl | G-6 | - | 151-152 |
| **97** | 3,5-Cl₂ | CF₃ | C | 2-Br | G-6 | - | 56-61 |
| **98** | 3,5-Cl₂ | CF₃ | C | 2-I | G-6 | - | NMR |
| **99** | 3,5-Cl₂ | CF₃ | C | 2-Me | G-6 | - | 49-56 |
| **100** | 3,5-Cl₂ | CF₃ | C | 2-CF₃ | G-6 | - | 54-59 |
| **103** | 3,5-Cl₂ | CF₃ | C | 2-OMe | G-6 | - | NMR |
| **104** | 3,5-Cl₂ | CF₃ | C | 2-NO₂ | G-6 | - | 64-72 |
| **105** | 3,5-Cl₂ | CF₃ | C | 2-NH₂ | G-6 | - | 244-246 |
| **106** | 3,5-Cl₂ | CF₃ | C | 2-NHC(O)Me | G-6 | - | 230-233 |
| **107** | 3,5-Cl₂ | CF₃ | C | 2-NHC(O)Et | G-6 | - | 199-201 |
| **108** | 3,5-Cl₂ | CF₃ | C | 2-NHC(O)Pr^{cyclo} | G-6 | - | |
| **109** | 3,5-Cl₂ | CF₃ | C | 2-NHCO₂Me | G-6 | - | 84-96 |
| **110** | 3,5-Cl₂ | CF₃ | C | 2-NHCO₂Et | G-6 | - | |
| **111** | 3,5-Cl₂ | CF₃ | C | 2-NHCO₂CH₂CCl₃ | G-6 | - | |
| **112** | 3,5-Cl₂ | CF₃ | C | 2-NHSO₂Me | G-6 | - | |
| **113** | 3,5-Cl₂ | CF₃ | C | 2-NHC(O)Ph | G-6 | - | 191-192 |
| **114** | 3,5-Cl₂ | CF) | N | - | G-6 | - | 161-163 |
| **115** | 3,5-Cl₂ | CF₃ | C | 2,6-F₂ | G-6 | - | NMR |
| **116** | 3-Br | CF₃ | C | - | G-6 | - | |
| **117** | 3-Br | CF₃ | C | 2-Br | G-6 | - | |
| **119** | 3,5-Br₂ | CF₃ | C | - | G-6 | - | |
| **120** | 3,5-Br₂ | CF₃ | C | 2-Br | G-6 | - | |
| **122** | 3-CF₃ | CF₃ | C | - | G-6 | - | 180-181 |
| **123** | 4-CF₃ | CF₃ | C | - | G-5 | - | 168-169 |
| **124** | 3-CF₃ | CF₃ | C | 2-Cl | G-6 | - | |
| **125** | 3-CF₃ | CF₃ | C | 2-Br | G-6 | - | 1.5609 (24°C) |
| **127** | 3-CF₃ | CF₃ | C | 2-NO₂ | G-6 | - | NMR |
| **128** | 3-CF₃ | CF₃ | C | 2-NH₂ | G-6 | - | |
| **129** | 3-CF₃ | CF₃ | C | 2-NHC(O)Me | G-6 | - | |
| **130** | 3-CF₃ | CF₃ | C | 2-NHC(O)Et | G-6 | - | |
| **131** | 3-CF₃ | CF₃ | C | 2-NHCO₂Me | G-6 | - | |
| **132** | 3,5-(CF₃)₂ | CF₃ | C | - | G-6 | - | NMR |
| **133** | 3,5-(CF₃)₂ | CF₃ | C | 2-Cl | G-6 | - | NMR |
| **134** | 3,5-(CF₃)₂ | CF₃ | C | 2-Br | G-6 | - | NMR |
| **136** | 3,5-(CF₃)₂ | CF₃ | C | 2-NO₂ | G-6 | - | NMR |
| **137** | 3,5-(CF₃)₂ | CF₃ | C | 2-NH₂ | G-6 | - | |
| **138** | 3,5-(CF₃)₂ | CF₃ | C | 2-NHC(O)Me | G-6 | - | |
| **139** | 3,5-(CF₃)₂ | CF₃ | C | 2-NHC(O)Et | G-6 | - | |
| **140** | 3,5-(CF₃)₂ | CF₃ | C | 2-NHCO₂Me | G-6 | - | |
| **141** | 3,5-Cl₂ | CF₃ | C | | G-7 | - | >250 |
| **142** | 3,5-Cl₂ | CF₃ | C | 2-Br | G-7 | - | |
| **144** | 3,5-F₂ | CF₃ | C | 2-NO₂ | G-8 | - | 58-63 |
| **145** | 3-Cl | CF₃ | C | 2-Br | G-8 | - | |
| **147** | 3-Cl | CF₃ | C | 2-NO₂ | G-8 | - | 103-107 |
| **148** | 3,5-Cl₂ | CF₃ | C | | G-8 | - | 198-199 (decomp.) |
| **149** | 3,5-Cl₂ | CF₃ | C | - | G-8 | 5-CF₃ | 147-151 |
| **150** | 3,5-Cl₂ | CF₃ | C | 2-Cl | G-8 | - | 1.5818 (24°C) |
| **151** | 3,5-Cl₂ | CF₃ | C | 2-Br | G-8 | - | 65-74 |
| **152** | 3,5-Cl₂ | CF₃ | C | 2-1 | G-8 | - | |
| **154** | 3,5-Cl₂ | CF₃ | C | 2-NO₂ | G-8 | - | NMR |
| **155** | 3,5-Cl₂ | CF₃ | C | 2-NO₂ | G-8 | 5-Me | 203-207 |
| **156** | 3,5-Cl₂ | CF₃ | C | 2-NO₂ | G-8 | 5-SMe | 156-159 (decomp.) |
| **157** | 3-CF₃ | CF₃ | C | | G-8 | - | |
| **158** | 3-CF₃ | CF₃ | C | 2-Cl | G-8 | - | |
| **159** | 3-CF₃ | CF₃ | C | 2-Br | G-8 | - | |
| **161** | 3-CF₃ | CF₃ | C | 2-NO₂ | G-8 | - | 58-66 |
| **162** | 3,5-(CF₃)₂ | CF₃ | C | | G-8 | - | |
| **163** | 3,5-(CF₃)₂ | CF₃ | C | 2-Cl | G-8 | - | |
| **164** | 3,5-(CF₃)₂ | CF₃ | C | 2-Br | G-8 | - | |
| **166** | 3,5-(CF₃)₂ | CF₃ | C | 2-NO₂ | G-8 | - | 173-175 |
| **167** | 3,5-F₂ | CF₃ | C | 2-NO₂ | G-9 | - | 166-168 (decomp.) |
| **168** | 3-Cl | CF₃ | C | 2-NO₂ | G-9 | - | 146-148 (decomp.) |
| **169** | 3,5-Cl₂ | CF₃ | C | - | -9 | - | |
| **170** | 3,5-Cl₂ | CF₃ | C | 2-Br | G-9 | - | |
| **172** | 3,5-Cl₂ | CF₃ | C | 2-NO₂ | G-9 | - | 54-63 |
| **173** | 3,5-Cl₂ | CF₃ | C | 2-NO₂ | G-9 | 4-Me | 144-147(decomp.) |
| **174** | 3,5-Cl₂ | CF₃ | C | 2-NO₂ | G-9 | 4-SMe | 54-60 |
| **176** | 3-CF₃ | CF₃ | C | 2-NO₂ | G-9 | - | 154-155 (decomp.) |
| **178** | 3,5-(CF₃)₂ | CF₃ | C | 2-NO₂ | G-9 | - | 147-149 |
| **179** | 3,4-Cl₂ | CF₃ | C | 2-Br | G-6 | - | 58-63 |

| | | | | | | | |
|---|---|---|---|---|---|---|---|
| NMR analysis | | | | | | | |

No.47
   ¹H-NMR (CDCl₃) δ: 3.81 (1H, d, *J* = 17.4 Hz), 4.24 (1H, d, *J* = 17.4 Hz), 7.94 (1H, d, *J* = 8.3 Hz), 8.00 (1H, s), 8.08-8.16 (4H, m), 8.38 (1H, s), 8.89 (1H, s).
No.80
   ¹H-NMR (CDCl₃) δ: 3.80 (1H, d, *J* = 17.2 Hz), 4.12 (1 H, d, *J* = 17.2 Hz), 7.50-7.58 (5H, m), 7.88-8.24 (4H, m), 8.87 (1H, s).
No.98
   ¹H-NMR (CDCl₃) δ: 3.72 (1H, d, *J* = 16.9 Hz), 4.10 (1H, d, *J* = 16.9 Hz), 7.47-7.84 (5H, m), 8.16 (1H, s), 8.26 (1H, s), 8.47 (1H, s).
No.103
   ¹H-NMR (CDCl₃) δ: 3.73 (1H, d, *J*= 17.2 Hz), 4.02 (3H, s), 4.12 (1H, d, *J* = 17.2 Hz), 7.21 (1H, dd, *J* = 8.2, 1.6 Hz), 7.44-7.59 (4H, m), 7.95 (1H, d, *J* = 8.2 Hz), 8.09 (1H, s), 8.89 (1H, s).
No.115
   ¹H-NMR (CDCl₃) δ: 3.70 (1H, d, *J* = 17.4 Hz), 4.12 (1H, d, *J* = 17.4 Hz), 7.23-7.58 (5H, m), 8.22 (1H, s), 8.40 (1H, s).
No.127
   ¹H-NMR (CDCl₃) δ: 3.81 (1H, d, *J* = 17.4 Hz),4.19 (1H, d, *J*= 17.4 Hz), 7.26-7.88 (5H, m), 8.09-8.24 (3H, m), 8.44 (1H, s).
No.132
   ¹H-NMR (CDCl₃) δ: 3.79 (1H, d, *J* = 17.2 Hz), 4.24 (1H, d, *J* = 17.2 Hz), 7.76-7.87 (4H, m), 7.97 (1H, d, *J*= 7.1 Hz), 8.12 (3H, d, *J* = 11.2 Hz), 8.62 (1H, t, *J*= 5.0 Hz).
No. 133
   ¹H-NMR (CDCl₃) δ: 3.94 (1H, d, *J* = 17.0 Hz), 4.42 (1H, d, *J* = 17.0 Hz), 7.67-8.14 (7H, m), 8.60 (1H, t, *J* = 7.9 Hz).
No.134
   ¹H-NMR (CDCl₃) δ: 3.78 (1H, d, *J* = 17.0 Hz), 4.22 (1H, d, *J* = 17.0 Hz), 7.59-8.16 (7H, m), 8.60 (1H, d, *J* = 3.3 Hz).
No.135
   ¹N-NMR (CDCl₃) δ: 3.81 (1H, d, *J* = 17.2 Hz), 4.24 (1H, d, *J* = 17.2 Hz), 7.79-8.14 (6H, m), 8.22 (1H, s), 8.90 (1H, s).
No.136
   ¹H-NMR (CDCl₃) δ: 3.83 (1H, d, *J* = 17.3 Hz), 4.24 (1H, d, *J* = 17.3 Hz), 7.74 (1H, d, *J* = 8.4 Hz), 8.01 (1H, s), 8.09-8.16 (4H, m), 8.24 (1H, d, *J* = 1.8 Hz), 8.45 (1H, s).
No.154
   ¹H-NMR (CDCl₃) δ: 3.81 (1H, d, *J* = 17.3 Hz), 4.17 (1H, d, *J* = 17.3 Hz), 7.46-7.52 (3H, m), 7.74 (1H, d, *J* = 8.1 Hz), 8.20 (1H, dd, *J* = 1.9, 8.2 Hz), 8.45 (1H, d, *J* = 1.9 Hz), 8.97 (1H, s).

**Table 2 (according to the invention)**

| | | | | | |
|---|---|---|---|---|---|
| 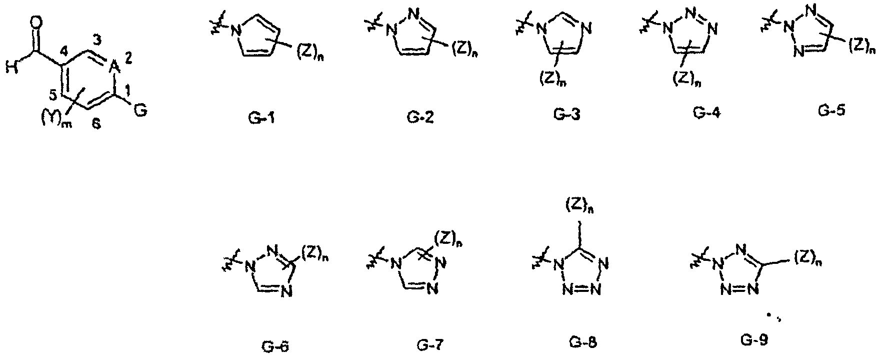 | | | | | |

| | A | (Y)ₘ | G | (Z)ₐ | melting point [°C] / refractive index |
|---|---|---|---|---|---|
| **3** | C | 2-CN | G-2 | 4-NO₂ | |
| **4** | C | 2-CN | G-2 | 4-CN | |
| **10** | C | 2-CN | G-6 | - | 134-141 |
| **12** | C | 2-CN | G-8 | - | |

**Table 2 (a illustrative example)**

| | | | | | |
|---|---|---|---|---|---|
| 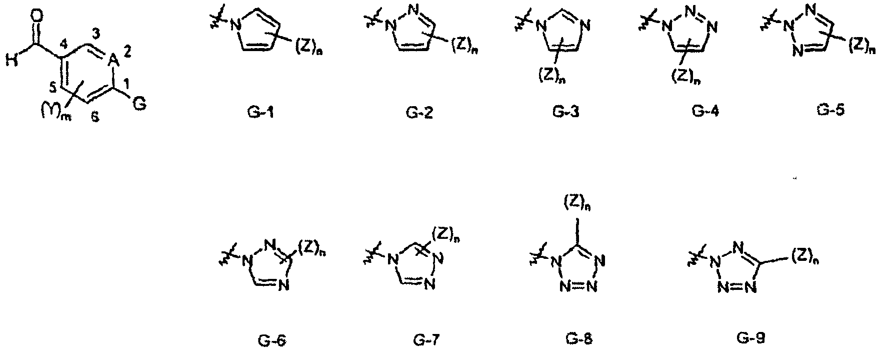 | | | | | |

| | A | (Y)ₘ | G | (Z)ₙ | melting point [°C] / refractive index |
|---|---|---|---|---|---|
| 1 | C | 2-Br | G-2 | 4-NO₂ | |
| 2 | C | 2-Br | G-2 | 4-CN | |
| 5 | C | - | G-6 | - | |
| 6 | C | -Cl | G-6 | - | 137-138 |
| 7 | C | 2-Br | G-6 | - | 123-129 |
| 8 | C | 2-Me | G-6 | - | 101-105 |
| 9 | C | 2-CF₃ | G-6 | - | 1.5343 (24 °C) |
| 11 | N | - | G-6 | - | 161-165 |

**Table 3 (according to the invention)**

| | | | | | |
|---|---|---|---|---|---|
| 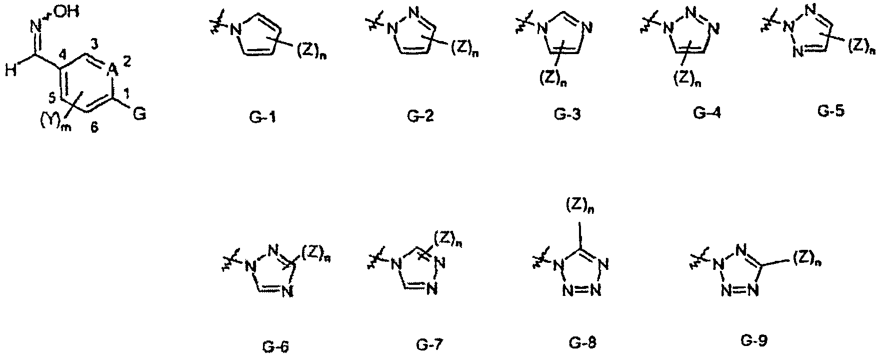 | | | | | |

| | A | (Y)ₘ | G | (Z)ₙ | melting point [°C] / refractive index |
|---|---|---|---|---|---|
| 3 | C | 2-CN | G-2 | 4-NO₂ | |
| 4 | C | 2-CN | G-2 | 4-CN | |
| 12 | C | 2-CN | G-8 | - | |

**Table 3 a (illustrative examples)**

| | | | | | |
|---|---|---|---|---|---|
| 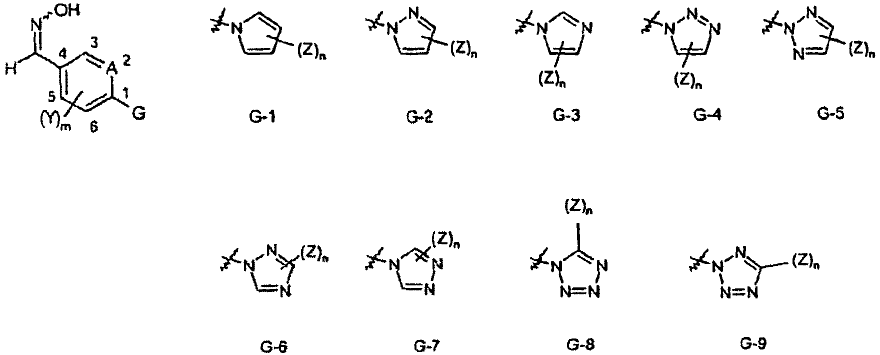 | | | | | |

| | A | (Y)ₘ | G | (Z)ₙ | melting point [°C] / refractive index |
|---|---|---|---|---|---|
| 1 | C | 2-Br | G-2 | 4-NO₂ | 229-232 |
| 2 | C | 2-Br | G-2 | 4-CN | |
| 5 | C | - | G-6 | - | 217-220 |
| 6 | C | -Cl | G-6 | - | >250 |
| 7 | C | 2-Br | G-6 | - | 157-163 |
| 8 | C | 2-Me | G-6 | - | 174-180 |
| 9 | C | 2-CF₃ | G-6 | - | 1.5242 (24°C) |
| 11 | N | - | G-6 | - | 221-223 |

**Table 4 (according to the invention)**

| | | | | | | |
|---|---|---|---|---|---|---|
| 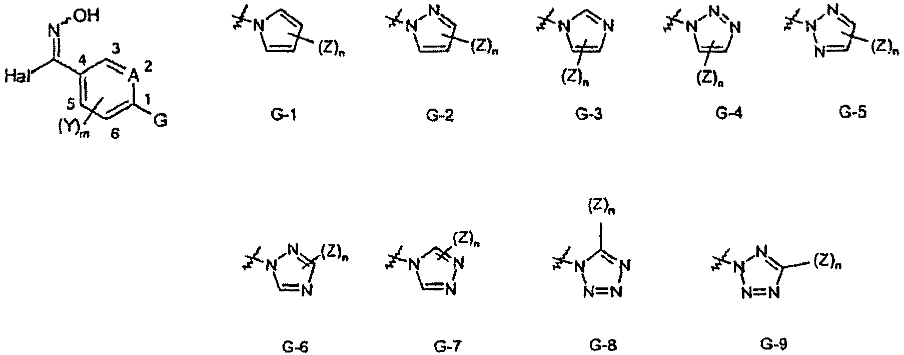 | | | | | | |

| | Hal | A | (Y)ₘ | G | (Z)ₙ | melting point [°C] / refractive index |
|---|---|---|---|---|---|---|
| | | | | | | |
| **3** | Cl | C | 2-CN | G-2 | 4-NO₂ | |
| **4** | Cl | C | 2-CN | G-2 | 4-CN | |
| **10** | Cl | C | 2-CN | G-6 | - | 184-185 |
| **12** | Cl | C | 2-CN | G-8 | - | |

**Table 4a (illustrative example)**

| | | | | | | |
|---|---|---|---|---|---|---|
| 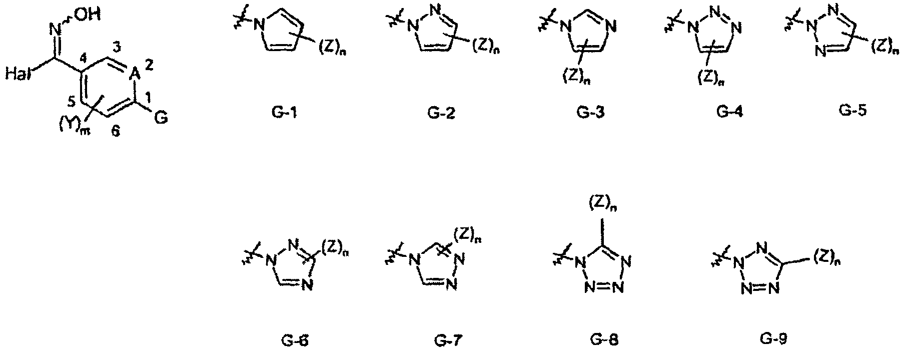 | | | | | | |

| | Hal | A | (Y)ₘ | G | (Z)ₙ | melting point [°C] / refractive index |
|---|---|---|---|---|---|---|
| **1** | Cl | C | 2-Br | G-2 | 4-NO₂ | |
| **2** | Cl | C | 2-Br | G-2 | 4-CN | |
| **5** | Cl | C | - | G-6 | - | |
| **6** | Cl | C | 2-Cl | G-6 | - | 152-157 |
| **7** | Cl | C | 2-Br | G-6 | - | |
| **8** | Cl | C | 2-Me | G-6 | - | |
| **9** | Cl | C | 2-CF₃ | G-6 | - | |
| **11** | Cl | N | - | G-6 | - | |

### Biological Test Example 1:

### Test for Spodoptera litura larvae

### Preparation of a test chemical solution

| | |
|---|---|
| Solvent: dimethylformamide | 3 parts by weight |
| Emulsifier: polyoxyethylene alkyl phenyl ether | 1 part by weight |

In order to obtain a preparation of a proper active compound, 1 part by weight of an active compound was mixed with the stated amounts of solvent and emulsifier and the mixture was diluted to a specified concentration with water.

### Test method

The leaves of sweat potatoes were dipped in the test solution diluted to a specified concentration with water. After these leaves were dried in air to remove the chemical solution, they were placed in a Petri dish of 9 cm in diameter, in which 10 Spodoptera litura larvae of the 3rd-instar were set free. The Petri dish was placed in a thermostatic chamber kept at 25°C and the leaves of sweat potatoes were added after 2 days and 4 days, to examine the number of dead larvae after 7 days, thereby calculating the insecticidal ratio of the chemical solution.

In this test, an average of the results in two Petri dishes in one section was calculated.

### Test results

In the above biological test 1, the above compound Nos. 2, 4, 5, 7, 8, 12, 13, 16, 17, 18, 19, 21, 23, 24, 25, 26, 27, 28, 29, 38, 47, 48, 52, 72, 86, 89, 90, 91, 93, 94, 96, 97, 98, 99, 100, 101, 103, 104, 105, 106, 107, 109, 114, 122, 123, 152, 126, 127, 132, 133, 134, 135, 136, 141, 148, 150, 151, 153, 154, 157, 160, 161, 162, 165, 166, 171, 176, 178 and 181, as typical examples, produced such a pest-controlling effect that the insecticidal ratio was 100% at an effective component concentration of 500 ppm.

### Biological Test Example 2:

### Test for Tetranychus urticae (spray test)

### Test method

50 to 100 adult Tetranychus urticae were inoculated on the leaves of a bean of two-leaf expansion period which was cultivated in a pot of 6 cm in diameter. After one day, the aqueous dilution containing the above prepared active compound having a specified concentration was sprayed on the leaves in a sufficient amount by a spray gun. After the spraying, the leaves were placed in a green house and the acaricidal ratio of the chemical solution was calculated after 7 days.

### Test results

The above compound Nos. 16, 21, 38, 80, 85, 89, 90, 94, 97, 98, 101, 103, 104, 109, 114, 123, 125, 126, 127, 132, 134, 135, 136, 150, 151, 153, 157, 160, 161, 165 and 181, as typical examples, produced such a pest-controlling effect that the acaricidal ratio was 98% or more at an effective component concentration of 100 ppm.

### Biological Test Example 3:

### Test for Aulacophora femoralis (spray test)

### Test method

The leaves of cucumbers were dipped in the aqueous solution diluted to a specified concentration with water. After the chemical solution was dried in air, these leaves were placed in a plastic cup, in which sterilized black soil was placed. 5 Aulacophora femoralis of the 2nd-instar were set free in the soil, to examine the number of dead insects after 7 days, thereby calculating the insecticidal ratio of the chemical solution.

### Test results

The above compound Nos. 21, 25, 38, 64, 80, 85, 86, 89, 90, 91, 94, 97, 98, 99, 100, 101, 103, 104, 106, 107, 109, 114, 122, 126, 127, 132, 134, 135, 136, 147, 148, 150, 151, 153, 154, 157, 160, 161, 165, 166, 171 and 181, as typical examples, produced such a pest-controlling effect that the insecticidal ratio was 100% at an effective component concentration of 500 ppm.

### Biological Test Example 4:

### Test for Myzus persicas resistant to organic phosphorous agents and carbamate agents

### Test method

About 200 head per plantlet of cultivated Myzus persicas resistant to phosphorous agents and carbamate agents were inoculated on an egg plant. The aqueous dilution containing the above prepared active compound having a specified concentration was sprayed on the egg plant in a sufficient amount by a spray gun one day after the inoculation. The egg plant was allowed to stand in a 28°C green house, to calculate the insecticidal ratio 24 hours after the solution was sprayed. In this case, the test was made twice repeatedly.

### Test results

The above compound Nos. 38, 101, 135 and 153, as typical examples, produced such a pest-controlling effect that the insecticidal ratio was 100% at an effective component concentration of 500 ppm.

### Biological Test Example 5:

### Test for Lucilia cuprina larvae

20 mg of active compound are dissolved in 1 ml of dimethyl sulphoxide. To prepare a suitable formulation (e.g. 100 ppm), the active compound solution is diluted to the respective desired concentration with water (e.g. 1 part by weight active compound solution with 199 parts by weight water).

Approximately 20 Lucilia cuprina larvae are introduced into a test tube which contains approx. 1 cm³ horse meat and 0.5 ml of the active compound preparation to be tested. After 48 hours, the efficacy of the active compound preparation is determined as % larval mortality. 0%: no larvae were killed, 100%: all larvae were killed.

In this test the above compounds nos. 8, 9, 12, 14, 16, 17, 18, 19, 20, 30, 36, 38, 40, 42, 43, 44, 45, 46, 54, 55, 58, 59, 60, 61, 62, 63, 66, 67, 68, 70, 80, 88, 89, 91, 93, 94, 76, 77, 78, 82, 71, 72, 73, 75, 84 showed a larval mortality of >95% at 100 ppm after 2 days.

### Biological test example 6:

### Test for Musca domestica

20 mg of the active compound are dissolved in 1 ml dimethylsulphoxide. To prepare a suitable formulation (e.g. 100 ppm), the active compound solution is diluted to the respective desired concentration with water (e.g. 1 part by weight active compound solution with 199 parts by weight water).

0.2 ml of this active compound preparation is pipetted onto a sponge (ca. ⊘ 1,5 cm) that has been wetted with 0.8 ml sugar solution. The sponge and 10 test animals are transferred to a dish (4x4 cm, h 2 cm) and covered.

After 48 hours the activity of the active compound preparation is determined. Here 100 % means that all flies were killed; 0 % means that no flies were killed.

In this test the above compounds nos. 8, 9, 14, 16, 18, 19, 20, 30, 36, 40, 42, 43, 44, 45, 46, 55, 58, 60, 61, 62, 66, 67, 68, 70, 80, 88, 89, 91, 93, 94, 76, 77, 78, 82, 71, 72, 73, 75, 84 showed a larval mortality of >95% at 100 ppm after 2 days.

### Biological test example 7:

### Test for Cat fleas/oral uptake

20 mg of the active compound are dissolved in 1 ml dimethylsulphoxide To prepare a suitable formulation (e.g. 100 ppm), the active compound solution is diluted to the respective desired concentration with cattle blood (e.g. 1 part by weight active compound solution with 199 parts by weight cattle blood).

20 unfed adult fleas (*Ctenocephalides felis,* strain "Georgi") are placed into a chamber (⊘ 5 cm) whose top and bottom are closed with gauze. A metal cylinder whose underside is covered with parafilm is placed onto the chamber. The cylinder contains the blood/active compound formulation which can be taken up by the fleas through the parafilm membrane. Whereas the blood is warmed to 37°C, the temperature in the area of the flea chambers is adjusted to room temperature.

After the desired time the mortality in % is determined. Here 100 % means that all fleas were killed; 0 % means that no fleas were killed.

In this test the above compounds nos. 8, 9, 12, 14, 16, 17, 18, 19, 20, 30, 36, 38, 40, 42, 43, 44, 54, 58, 60, 61, 62, 66, 67, 80, 88, 89, 91, 93, 94, 76, 77, 78, 82, 71, 72, 73, 75, 84 effected a > 80% kill at 100 ppm after 2 days.

### Biological test example 8:

### Rhipicephalus (Boophilus) microplus; injection

20 mg of active compound are dissolved in 1 ml of dimethyl sulphoxide, lower concentrations are prepared by dilution in the same solvent.

The test is carried out in quintuplicates with female fully engorged cattle ticks collected no later than 24 hrs after drop-off from their host. 1 µl of the solutions is injected into the abdomen, and the ticks are transferred into replica dishes and stored in a controlled-environment chamber. The activity is checked after 7 days for the deposit of fertile eggs. Eggs whose fertility is not externally discernible are stored in glass tubes in a controlled-environment cabinet until larvae hatch after approximately 42 days. 100% activity means that no tick has laid fertile eggs.

In this test the above compounds nos. 8, 9, 12, 14, 16, 17, 18, 20, 30, 36, 38, 40, 42, 43, 44, 45, 46, 63, 66, 67, 68, 70, 80, 88, 89, 91, 93, 94, 76, 77, 78, 82, 71, 72, 73, 75, 84 effected > 90% activity at 20 µg/tick after 7 days.

### Preparation Example I (granule)

25 parts of water was added to a mixture of 10 parts of the compound (No. 26) of the present invention, 30 parts of bentonite (montmorillonite), 58 parts of talc and 2 parts of lignin sulfonate and the resulting mixture was sufficiently kneaded and granulated into a 10 to 40 mesh granular form by an extrusion type granulator, followed by drying at 40 to 50°C to form granules.

### Preparation Example 2 (granule)

95 parts of clay mineral particles having a grain size distribution ranging from 0.2 to 2 mm was put into a rotary mixer. 5 parts of the compound (No. 72) of the present invention was sprayed together with a liquid diluent to humidify the particles uniformly with rotating the mixer and then dried at 40 to 50°C to form granules.

### Preparation Example 3 (emulsifiable concentrate)

30 parts of the compound (No. 107) of the present invention, 55 parts of xylene, 8 parts of polyoxyethylene alkyl phenyl ether and 7 parts of calcium alkylbenzenesulfonate were mixed and stirred to prepare emulsifiable concentrates.

### Preparation Example 4 (wettable powder)

15 parts of the compound (No. 91) of the present invention, 80 parts of a mixture of white carbon (hydrous amorphous silicon oxide powder) and powdery clay (1 : 5), 2 parts of sodium alkylbenzenesulfonate and 3 parts of sodium alkylnaphthalenesulfonate-formalin condensate were pulverized and mixed to prepare wettable powders.

### Preparation Example 5 (dry flowables)

20 parts of the compound (No. 114) of the present invention, 30 parts of sodium lignin sulfonate, 15 parts of bentonite and 35 parts of calcined diatomaceous earth were thoroughly mixed. Water was added to the mixture, which was then extruded through a 0.3 mm screen and dried to form dry flowables.

## Claims

1. Isoxazolines of the formula (I) 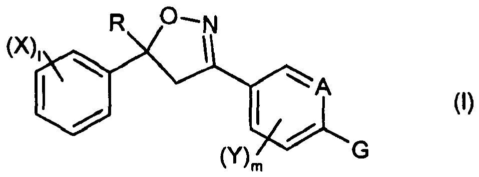 wherein:
A represents C or N;
R represents C₁₋₄ haloalkyl;
X represents the same or different halogen or C₁₋₄ haloalkyl;
l represents 0, 1 or 2;
Y represents cyano;
m represents 1 or 2; and
G represents a heterocyclic group, selected from the group consisting of the following groups G-1 to G-9: 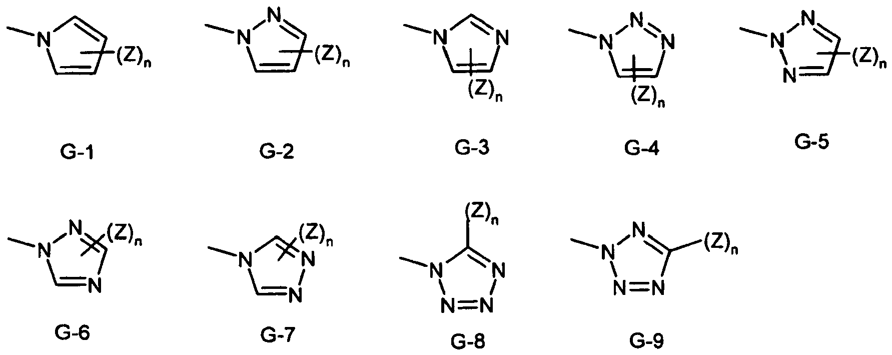
wherein
Z represents halogen, methyl, methylthio, trifluoromethyl, cyano, nitro or amino; and
n represents 0 or 1.

2. Compounds according to Claim 1, wherein
A represents C or N;
R represents trifluoromethyl or pentafluoroethyl;
X represents, independently of each other, fluoro, chloro, bromo or trifluoromethyl;
l represents 0, 1 or 2;
Y cyano,;
m represents 1 or 2; and
Gis selected from heterocyclic groups represented by the formulae G-1 to G-9: 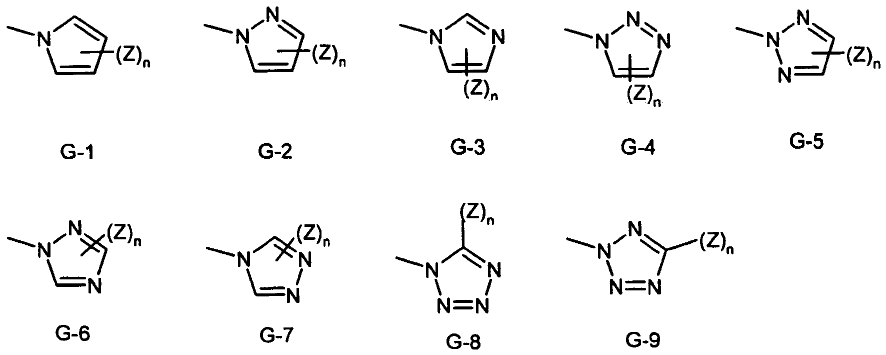 wherein
Z represents halogen, methyl, methylthio, trifluoromethyl, cyano, nitro or amino; and
n represents 0 or 1.

3. Composition, comprising at least one compound of formula (I) according to claim 1 or 2 for controlling harmful insects.

4. Use of a compound or composition as defined in any one of claims 1 to 3 for controlling harmful insects.

5. Use of a compound as defined in any of claim 1 or 2 for preparing a composition for controlling animal parasites.

6. Use according to claim 5 wherein the animal parasites are parasitic arthropods.

7. Process for the preparation of compounds of formula (I) as defined in claim 1, **characterized in that**
a compound of formula (II) 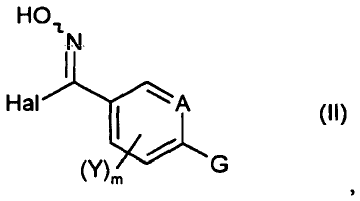 wherein A, Y, m and G have the meaning as defined in claim 1 and Hal represents halogen, is reacted with a compound of formula (III) 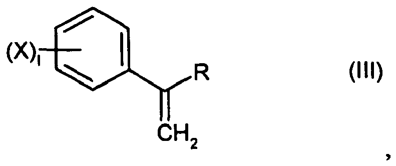 wherein R, X and I have the same meaning defined in claim 1,
in the presence of inert solvents, and optionally in the presence of a base,
or
a compound of formula (IV) 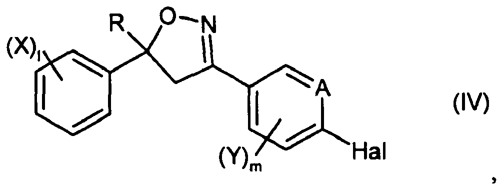 wherein A, R, X, 1, Y, and m have the meaning as defined in claim 1, and Hal represents halogen, are reacted with compounds of formula (V)
G-H (V)
wherein G has the same meaning as defined in claim 1,
in the presence of inert solvents, and optionally in the presence of a base.

8. Process for the preparation of compounds of formula (I), as defined in claim 1, wherein G represents 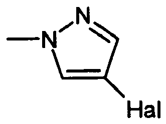 wherein Hal represent halogen, **characterized in that** compounds of the formula (Ia) 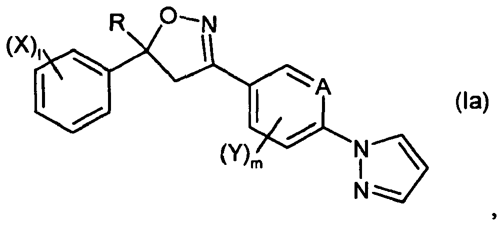 wherein A, R, X, 1, Y and m have the meaning as defined in claim 1, are reacted with halogenating agents in the presence of inert solvents.

9. Process for the preparation of compounds of formula (I) as defined in claim 1, wherein G
represents 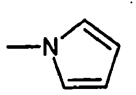 **characterized in that** compounds of formula (VI) 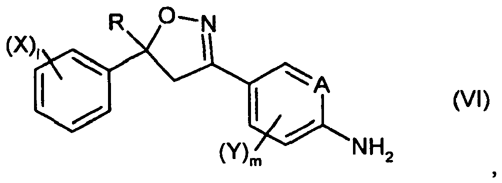 wherein A, R X, 1, Y and m have the meaning as defined in claim 1,
are reacted with compounds of formula (VII) 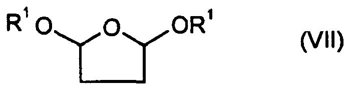 wherein R¹ represents alkyl,
in the presence of inert solvents.

10. Process for the preparation of compounds of formula (I) as defined in claim 1, wherein G represents 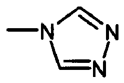 **characterized in that** the compounds of formula (VI) as defined in claim 9 are reacted with 1,2-diformylhydrazine in the presence of a base.

11. Process for the preparation of compounds of formula (I) as defined in claim 1, wherein G represents 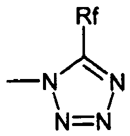 wherein Rf represents perfluoroalkyl, **characterized in that** compounds of the formula (VIII) 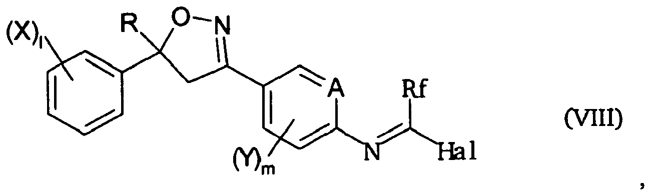 wherein A, R, X, 1, Y and m, have the meaning as defined in claim 1 and Hal represent halogen, are reacted with azide compounds in the presence of inert solvents.

12. Process for the preparation of compounds of formula (I) as defined in claim 1, wherein G represents 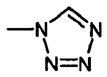 **characterized in that** compounds of formula (VI) as defined in claim 9 are reacted with azide compounds and trialkyl orthoformates in the presence of inert solvents.

13. Process for the preparation of compounds of formula (I) as defined in claim 1, wherein A represents C and at least one of (Y)ₘ represents 3-NH_{2.} **characterized in that** compounds of the formula (Ib) 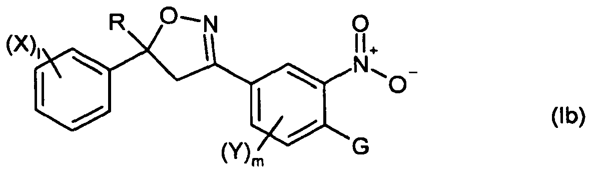 wherein R, X, 1, Y, m and G have the meaning as defined in claim 1, are reduced in the presence of inert solvents.

14. Process for the preparation of compounds of formula (I) as defined in claim 1, wherein A represents C and at least one of (Y)ₘ represents 3-NH-R², in which R² represents acyl, alkoxycarbonyl, haloalkoxycarbonyl or alkylsulfonyl, **characterized in that** compounds of the formula (Ic) 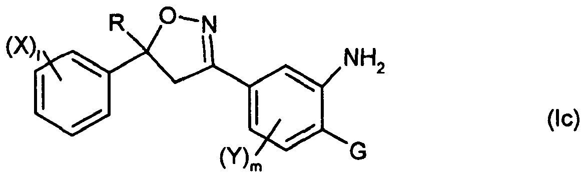 wherein R, X, 1, Y, m and G have the meaning as defined in claim 1 are reacted with compounds of the formula (IX)
R²-T (IX)
wherein T represents halogen or hydroxy, in the presence of inert solvents, and optionally in the presence of a base.

15. Compounds usable for the preparation of compounds of formula (I) as defined in claim 1 having the following formula 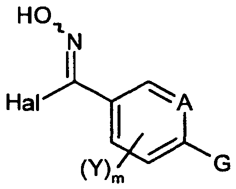 wherein A, Y, m and G have the meaning as defined in Claim 1, and Hal represents halogen.

16. Compounds usable for the preparation of compounds of formula (I) as defined in claim 1 having the following formula 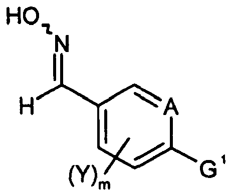 wherein A, Y m, and G¹ have the meaning as defined in Claim I, provided that when A represents C and m represents 0, G¹ is not 1H-imidazol-1-yl.

17. Compounds usable for the preparation of compounds of formula (I) as defined in claim 1 having the following formula 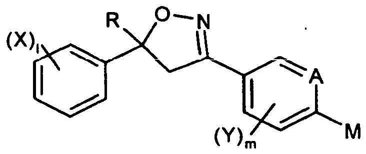 wherein A, R, X, I, Y and m have the meaning as defined in Claim 1 and M represents a group: 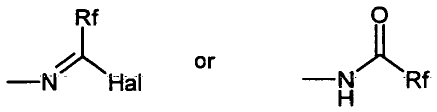 in which Hal represents halogen, and Rf represents perfluoroalkyl.

## Patentansprüche

1. Isoxazoline der Formel (I) 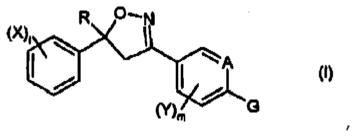 wobei:
A für C oder N steht;
R für C₁₋₄-Halogenalkyl steht;
X für gleiche oder unterschiedliche Halogen- oder C₁₋₄-Halogenalkylgruppen steht;
l für 0, 1 oder 2 steht;
Y für Cyano steht;
m für 1 oder 2 steht und
G für eine aus der aus den folgenden Gruppen G-1 bis G-9 bestehenden Gruppe ausgewählte heterocyclische Gruppe steht: 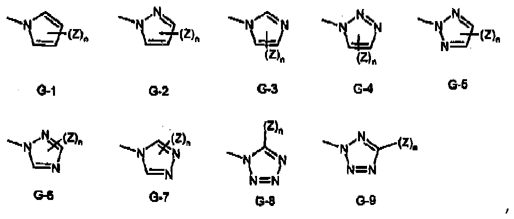
wobei
Z für Halogen, Methyl, Methylthio, Trifluormethyl, Cyano, Nitro oder Amino steht und
n für 0 oder 1 steht.

2. Verbindungen nach Anspruch 1, wobei
A für C oder N steht;
R für Trifluormethyl oder Pentafluorethyl steht;
X unabhängig von den anderen Resten X für Fluor, Chlor, Brom oder Trifluormethyl steht;
l für 0, 1 oder 2 steht;
Y für Cyano steht;
m für 1 oder 2 steht und
G aus den durch die Formeln G-1 bis G-9 wiedergegebenen heterocyclischen Gruppen ausgewählt ist; 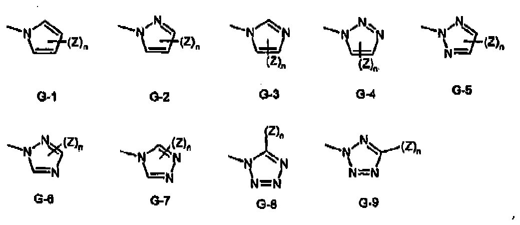
wobei
Z für Halogen, Methyl, Methylthio, Trifluormethyl, Cyano, Nitro oder Amino steht und
n für 0 oder 1 steht.

3. Zusammensetzung, enthaltend mindestens eine Verbindung der Formel (I) nach Anspruch 1 oder 2 zur Bekämpfung von Schadinsekten.

4. Verwendung einer wie in einem der Ansprüche 1 bis 3 definierten Verbindung oder Zusammensetzung zur Bekämpfung von Schadinsekten.

5. Verwendung einer wie in Anspruch 1 oder 2 definierten Verbindung zur Herstellung einer Zusammensetzung zur Bekämpfung von tierischen Parasiten.

6. Verwendung nach Anspruch 5, wobei es sich bei den tierischen Parasiten um parasitische Arthropoden handelt.

7. Verfahren zur Herstellung von wie in Anspruch 1 definierten Verbindungen der Formel (I), **dadurch gekennzeichnet, dass** man eine Verbindung der Formel (II) 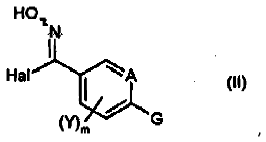 in welcher A, Y, m und G die wie in Anspruch 1 definierte Bedeutung haben und Hal für Halogen steht, mit einer Verbindung der Formel (III) 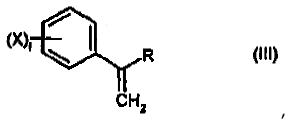 in welcher R, X und 1 die gleiche, in Anspruch 1 definierte Bedeutung haben,
in Gegenwart von inerten Lösungsmitteln und gegebenenfalls in Gegenwart einer Base umsetzt, oder
eine Verbindung der Formel (IV) 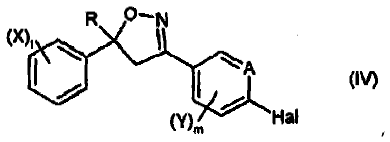 in welcher A, R, X, 1, Y und m die wie in Anspruch 1 definierte Bedeutung haben und Hal für Halogen steht, mit Verbindungen der Formel (V)
G-H, (V)
in welcher G die gleiche, wie in Anspruch 1 definierte Bedeutung hat,
in Gegenwart von inerten Lösungsmitteln und gegebenenfalls in Gegenwart einer Base umsetzt.

8. Verfahren zur Herstellung von wie in Anspruch 1 definierten Verbindungen der Formel (I), wobei G für 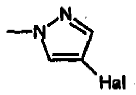 steht, wobei Hal für Halogen steht, **dadurch gekennzeichnet, dass** man Verbindungen der Formel (Ia) 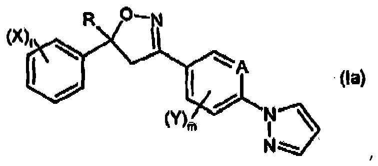 in welcher A, R, X, 1, Y und m die wie in Anspruch 1 definierte Bedeutung haben, in Gegenwart von inerten Lösungsmitteln mit Halogenierungsmitteln umsetzt.

9. Verfahren zur Herstellung von wie in Anspruch 1 definierten Verbindungen der Formel (I), wobei G für 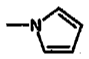 steht, **dadurch gekennzeichnet, dass** man Verbindungen der Formel (VI) 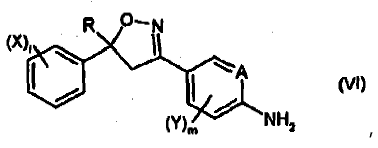 in welcher A, R, X, 1, Y und m die wie in Anspruch 1 definierte Bedeutung haben,
mit Verbindungen der Formel (VII) 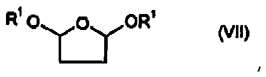 in welcher R¹ für Alkyl steht, in Gegenwart von inerten Lösungsmitteln umsetzt.

10. Verfahren zur Herstellung von wie in Anspruch 1 definierten Verbindungen der Formel (I), wobei G für 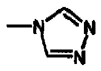 steht,
**dadurch gekennzeichnet, dass** man die wie in Anspruch 9 definierten Verbindungen der Formel (VI) in Gegenwart einer Base mit 1,2-Diformylhydrazin umsetzt.

11. Verfahren zur Herstellung von wie in Anspruch 1 definierten Verbindungen der Formel (I), wobei G für 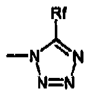 steht, wobei Rf für Perfluoralkyl steht, **dadurch gekennzeichnet, dass** man Verbindungen der Formel (VIII) 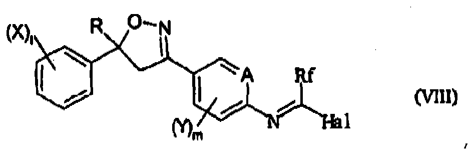 in welcher A, R, X, l, Y und m die wie in Anspruch 1 definierte Bedeutung haben und Hal für Halogen steht, in Gegenwart von inerten Lösungsmitteln mit Azidverbindungen umsetzt.

12. Verfahren zur Herstellung von wie in Anspruch 1 definierten Verbindungen der Formel (I), wobei G für 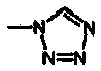 steht, **dadurch gekennzeichnet, dass** man wie in Anspruch 9 definierte Verbindungen der Formel (VI) in Gegenwart von inerten Lösungsmitteln mit Azidverbindungen und Orthoameisensäuretrialkylestern umsetzt.

13. Verfahren zur Herstellung von wie in Anspruch 1 definierten Verbindungen der Formel (I), wobei A für C steht und mindestens ein (Y)ₘ für 3-NH₂ steht, **dadurch gekennzeichnet, dass** man Verbindungen der Formel (Ib) 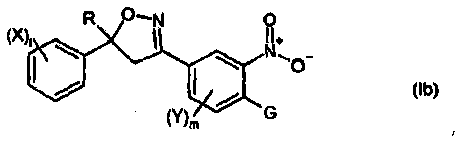 in welcher R, X, l, Y, m und G die wie in Anspruch 1 definierte Bedeutung haben, in Gegenwart von inerten Lösungsmitteln reduziert.

14. Verfahren zur Herstellung von wie in Anspruch 1 definierten Verbindungen der Formel (I), wobei A
für C steht und mindestens ein (Y)ₘ für 3-NH-R² steht, wobei R² für Acyl, Alkoxycarbonyl, Halogenalkoxycarbonyl oder Alkylsulfonyl steht, **dadurch gekennzeichnet, dass** man Verbindungen der Formel (Ic) 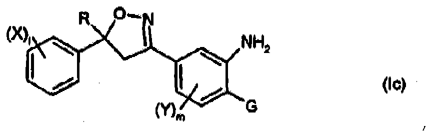 in welcher R, X, l, Y, m und G die wie in Anspruch 1 definierte Bedeutung haben, in Gegenwart von inerten Lösungsmitteln und gegebenenfalls in Gegenwart einer Base mit Verbindungen der Formel (IX) umsetzt,
R²-T (IX)
wobei T für Halogen oder Hydroxy steht.

15. Für die Herstellung von wie in Anspruch 1 definierten Verbindungen der Formel (I) geeignete Verbindungen mit der folgenden Formel 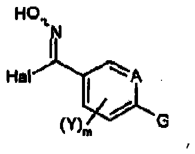 wobei A, Y, m und G die wie Anspruch 1 definierte Bedeutung haben und Hal für Halogen steht.

16. Für die Herstellung von wie in Anspruch 1 definierten Verbindungen der Formel (I) geeignete Verbindungen mit der folgenden Formel 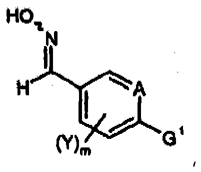 wobei A, Y, m und G¹ die wie Anspruch 1 definierte Bedeutung haben, mit der Maßgabe, dass, wenn A für C steht und m für 0 steht, G¹ nicht für 1H-Imidazol-1-yl steht.

17. Für die Herstellung von wie in Anspruch 1 definierten Verbindungen der Formel (I) geeignete Verbindungen mit der folgenden Formel 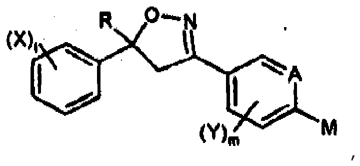 wobei A, R, X, 1, Y und m die wie Anspruch 1 definierte Bedeutung haben und M für eine Gruppe: 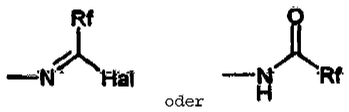 steht, wobei Hal für Halogen steht und Rf für Perfluoralkyl steht.

## Revendications

1. Isoxazolines de formule (I) 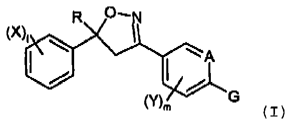 dans laquelle
A représente C ou N ;
R représente halogénoalkyle en C₁₋₄ ;
X représente un halogène ou halogénoalkyle en C₁₋₄ identique ou différent ;
l eprésente 0, 1 ou 2 ;
Y représente cyano
m représente 1 ou 2 ; et
G représente un groupe hétérocyclique, choisi dans le groupe constitué des groupes suivants G-1 à G-9 : 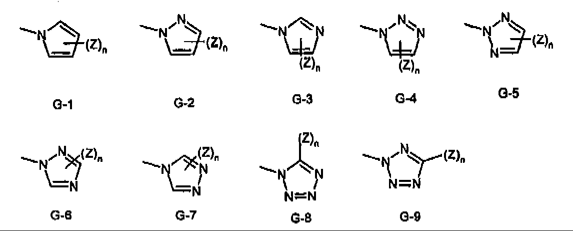
dans lesquels
Z représente halogène, méthyle, méthylthio, trifluorométhyle, cyano, nitro ou amino ; et
n représente 0 ou 1.

2. Composés selon la revendication 1, dans lesquels
A représente C ou N ;
R représente trifluorométhyle ou pentafluoroéthyle ;
X représente, indépendamment les uns des autres, fluoro, chloro, bromo ou trifluorométhyle ;
l eprésente 0, 1 ou 2 ;
Y est cyano ;
m représente 1 ou 2 ; et
G est choisi parmi des groupes hétérocycliques représentés par les formules G-1 à G-9 ; 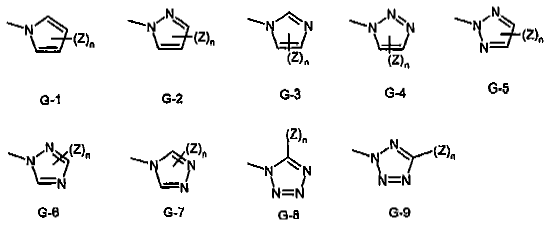
dans lesquelles
Z représente halogène, méthyle, méthylthio, trifluorométhyle, cyano, nitro ou amino ; et
n représente 0 ou 1.

3. Composition comprenant au moins un composé de formule (I) selon la revendication 1 ou 2 pour lutter contre des insectes nuisibles.

4. Utilisation d'un composé ou une composition tels que définis dans l'une quelconque des revendications 1 à 3 pour lutter contre des insectes nuisibles.

5. Utilisation d'un composé tel que défini dans l'une quelconque des revendications 1 ou 2 pour préparer une composition pour lutter contre des parasites animaux.

6. Utilisation selon la revendication 5 dans laquelle les parasites animaux sont des arthropodes parasitaires.

7. Procédé pour la préparation de composés de formule (I) tels que définis dans la revendication 1, **caractérisé en ce que**
un composé de formule (II) 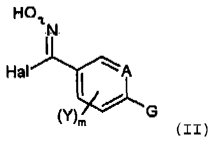 dans laquelle A, Y, m et G ont la signification telle que définie dans la revendication 1 et Hal représente halogène,
réagit avec un composé de formule (III) 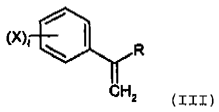 dans laquelle R, X et 1 ont la même signification que celle définie dans la revendication 1,
en présence de solvants inertes, et facultativement en présence d'une base,
ou
un composé de formule (IV) 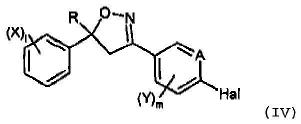 dans laquelle A, R, X, 1, Y et m ont la signification telle que définie dans la revendication 1, et Hal représente halogène, réagit avec des composés de formule (V)
G-H (V)
dans laquelle G a la même signification que celle définie dans la revendication 1,
en présence de solvants inertes, et facultativement en présence d'une base.

8. Procédé pour la préparation de composés de formule (I), tels que définis dans la revendication 1, dans lesquels G représente 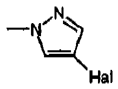 dans lequel Hal représente halogène, **caractérisé en ce que** des composés de formule (Ia) 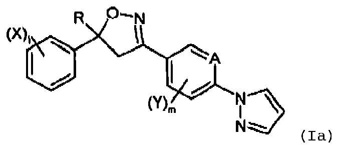 dans laquelle A, R, X, 1, Y et m ont la signification telle que définie dans la revendication 1, réagissent avec des agents d'halogénation en présence de solvants inertes.

9. Procédé pour la préparation de composés de formule (I) tels que définis dans la revendication 1, dans lesquels G représente 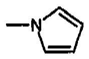 **caractérisé en ce que** des composés de formule (VI) 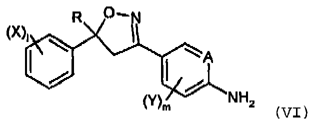 dans laquelle A, R, X, 1, Y et m ont la signification telle que définie dans la revendication 1,
réagissent avec des composés de formule (VII) 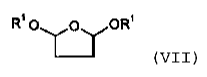 dans laquelle R¹ représente alkyle,
en présence de solvants inertes.

10. Procédé pour la préparation de composés de formule (I) tels que définis dans la revendication 1, dans lesquels G représente 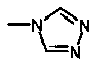 **caractérisé en ce que** les composés de formule (VI) tels que définis dans la revendication 9 réagissent avec de la 1,2-diformylhydrazine en présence d'une base.

11. Procédé pour la préparation de composés de formule (I) tels que définis dans la revendication 1, dans lesquels G représente 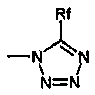 dans lequel Rf représente perfluoroalkyle, **caractérisé en ce que** des composés de formule (VIII) 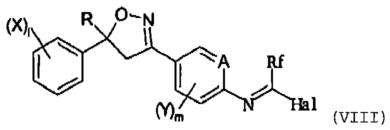 dans laquelle A, R, X, L, Y et m ont la signification telle que définie dans la revendication 1 et Hal représente halogène, réagissent avec des composés d'azide en présence de solvants inertes.

12. Procédé pour la préparation de composés de formule (I) tels que définis dans la revendication 1, dans lesquels G représente 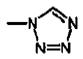 **caractérisé en ce que** des composés de formule (VI) tels que définis dans la revendication 9 réagissent avec des composés d'azide et des orthoformiates de trialkyle en présence de solvants inertes.

13. Procédé pour la préparation de composés de formule (I) tels que définis dans la revendication 1, dans lesquels A représente C et au moins l'un de (Y)ₘ représente 3-NH₂, **caractérisé en ce que** des composés de formule (Ib) 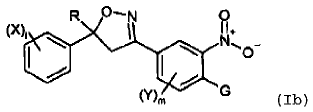 dans lesquels R, X, 1, Y, m et G ont la signification telle que définie dans la revendication 1, sont réduits en présence de solvants inertes.

14. Procédé pour la préparation de composés de formule (I) tels que définis dans la revendication 1, dans lequel A représente C et au moins l'un de (Y)ₘ représente 3-NH-R², dans lequel R² représente acyle, alcoxycarbonyle, halogénoalcoxycarbonyle ou alkylsulfonyle, **caractérisé en ce que** des composés de formule (IC) 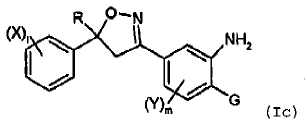 dans laquelle R, X, 1, Y, m et G ont la signification telle que définie dans la revendication 1 réagissent avec des composés de formule (IX)
R²-T (IX)
dans laquelle T représente halogène ou hydroxy, en présence de solvants inertes, et facultativement en présence d'une base.

15. Composés utilisables pour la préparation de composés de formule (I) tels que définis dans la revendication 1 ayant la formule suivante 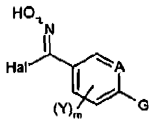 dans laquelle A, Y, m et G ont la signification telle que définie dans la revendication 1, et Hal représente halogène.

16. Composés utilisables pour la préparation de composés de formule (I) tels que définis dans la revendication 1 ayant la formule suivante 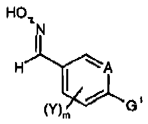 dans laquelle A, Y, m et G¹ ont la signification telle que définie dans la revendication 1, à condition que lorsque A représente C et m représente 0, G¹ ne soit pas 1H-imidazol-1-yle.

17. Composés utilisables pour la préparation de composés de formule (I) tels que définis dans 1a revendication 1 ayant la formule suivante 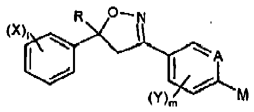 dans laquelle A, R, X, 1, Y et m ont la signification telle que définie dans la revendication 1, et M représente un groupe : 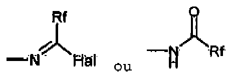 dans lesquels Hal représente halogène, et Rf représente perfluoroalkyle,
